# FASCICULE DE BREVET EUROPEEN

(11) **EP 0 919 541 B1**
(45) Date de publication et mention de la délivrance du brevet: **10.08.2005**
(21) Numéro de dépôt: 98402963.7
(22) Date de dépôt: 27.11.1998
(51) Int. Cl.: C07C 233/22, C07C 233/05, C07C 233/60, C07C 233/58, C07C 233/31, A61K 31/16, C07C 309/65, C07D 307/12, C07D 333/16

(54) **Nouveaux composés naphtaléniques, leur procédé de préparation et les compositions pharmaceutiques qui les contiennent**
Naphthalen Verbindungen, Verfahren zu deren Herstellung und deren pharmazeutischen Zusammensetzungen
Naphthalene compounds, process for their preparation and their pharmaceutical compositions

(30) Priorité: 28.11.1997 FR 9714975
(43) Date de publication de la demande: 02.06.1999
(73) Titulaire: LES LABORATOIRES SERVIER, 92200 Neuilly sur Seine (FR)
(72) Inventeur: Lefoulon, Francois, 45000 Orleans (FR); Demuynck, Luc, 45000 Orleans (FR); Lesieur, Daniel, 59147 Gondecourt (FR); Depreux, Patrick, 59280 Armentieres (FR); Bennejean, Caroline, 94220 Charenton le Pont (FR); Renard, Pierre, 78150 Le Chesnay (FR); Delagrange, Philippe, 92130 Issy les Moulineaux (FR)

(56) Documents cités:
- EP-A- 0 662 471
- EP-A- 0 721 938
- EP-A- 0 728 738
- EP-A- 0 745 583
- EP-A- 0 745 584
- EP-A- 0 745 586
- WO-A-96/08466
- US-A- 3 467 710

## Description

La présente invention concerne de nouveaux dérivés à structure naphtalénique, leur procédé de préparation, et les compositions pharmaceutiques qui les contiennent.

Les brevets EP 447285, EP 530087 et EP 562956 décrivent des dérivés naphtylalkylamide, naphtylakylurée et naphtylalkylthiourée possédant des propriétés pharmacologiques intéressantes grâce à leur affinité pour les récepteurs de la mélatonine et à leur caractère agoniste ou antagoniste. Tous ces dérivés se caractérisent par la présence d'un seul substituant sur le noyau portant la chaîne alkylamide ou alkylurée. La demande WO 9706140 décrit des ligands mélatoninergiques de nature voisine, acylés sur le noyau principal porteur de la chaîne alkylamide.

De nombreuses études ont mis en évidence ces dix dernières années le rôle capital de la mélatonine (N-acétyl-5-méthoxytryptamine) dans de nombreux phénomènes physiopathologiques ainsi que dans le contrôle du rythme circadien. Toutefois, elle possède un temps de demi-vie assez faible dû à une rapide métabolisation. Il est donc très intéressant de pouvoir mettre à la disposition du clinicien des analogues de la mélatonine, métaboliquement plus stables et présentant un caractère agoniste ou antagoniste, dont on peut attendre un effet thérapeutique supérieur à celui de l'hormone elle-même.

Outre leur action bénéfique sur les troubles du rythme circadien (J. Neurosurg. 1985, 63, pp 321-341) et du sommeil (Psychopharmacology, 1990, 100, pp 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp 264-272) et analgésiques (Pharmacopsychiat., 1987, 20, pp 222-223) ainsi que pour le traitement des maladies de Parkinson (J. Neurosurg. 1985, 63, pp 321-341) et d'Alzheimer (Brain Research, 1990, 528, pp 170-174). De même, ces composés ont montré une activité sur certains cancers (Melatonin - Clinical Perspectives, Oxford University Press, 1988, pp 164-165), sur l'ovulation (Science 1987, 227, pp 714-720), sur le diabète (Clinical Endocrinology, 1986, 24, pp 359-364), et sur le traitement de l'obésité (International Journal of Eating Disorders, 1996, 20 (4), pp 443-446).

Ces différents effets s'exercent par l'intermédiaire de récepteurs spécifiques de la mélatonine. Des études de biologie moléculaire ont montré l'existence de plusieurs sous-types réceptoriels pouvant lier cette hormone (Trends Pharmacol Sci., 1995, 16, p 50 ; WO 97.04094). Certains de ces récepteurs ont pu être localisés et caractérisés pour différentes espèces, dont les mammifères. Afin de pouvoir mieux comprendre les fonctions physiologiques de ces récepteurs, il est d'un grand intérêt de disposer de ligands spécifiques. De plus, de tels composés, en interagissant sélectivement avec l'un ou l'autre de ces récepteurs, peuvent être pour le clinicien d'excellents médicaments pour le traitement des pathologies liées au système mélatoninergique, dont certaines ont été mentionnées précédemment.

Des dérivés 1-naphtylalkylamide substitués en position 3 par un reste aralkyle et ayant une affinité pour les récepteurs de la mélatonine, ont été divulgués dans le brevet EP 662471.

Les composés de la présente invention présentent une structure originale se caractérisant par un noyau naphtylalkylamide, -urée, partiellement hydrogéné ou non et présentant deux substituants sur le noyau principal. Cette structure leur confère de façon surprenante une très grande affinité pour les récepteurs de la mélatonine et une sélectivité pour l'un ou l'autre des sous-types réceptoriels.

L'invention concerne les composés de formule (I) : 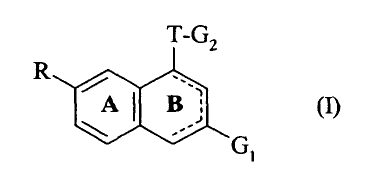 dans laquelle :
- les cycles A et B forment ensemble un groupement naphtalène, 1,2-dihydronaphtalène, 2,3-dihydronaphtalène, 1,4-dihydronaphtalène, ou tétrahydronaphtalène,
- T représente une chaîne alkylène (C₁-C₆) linéaire ou ramifiée éventuellement substituée par un ou plusieurs groupements alkyle (C₁-C₆) linéaire ou ramifié, hydroxy, alkoxy (C₁-C₆) linéaire ou ramifié, carboxy, ou alkoxycarbonyle (C₁-C₆) linéaire ou ramifié,
- R représente un radical R' ou un groupement OR', R' représentant un groupement alkyle (C₁-C₆) linéaire ou ramifié, ou alkényle (C₂-C₆) linéaire ou ramifié,
- G₁ représente un groupement aryle éventuellement substitué, ou hétéroaryle éventuellement substitué,
- G₂ représente un groupement choisi parmi : 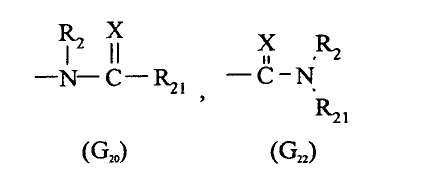 dans lesquels :
   - X représente un atome d'oxygène,
   - R₂ représente un atome d'hydrogène,
   - R₂₁ représente un groupement alkyle (C₁-C₆) linéaire ou ramifié éventuellement substitué par un ou plusieurs atomes d'halogène, alkényle (C₂-C₆) linéaire ou ramifié, alkynyle (C₂-C₆) linéaire ou ramifié, ou cycloalkyle (C₃-C₇),
   étant entendu que :
   - lorsque G₂ représente un groupement G₂₂, alors G₁ représente un groupement, hétéroaryle (différent d'un pyridyle) éventuellement substitué,
   - lorsque G₂ représente un groupement G₂₀ et G₁ est différent d'un groupement hétéroaryle éventuellement substitué, alors T représente une chaîne méthylène ou éthylène éventuellement substituée par un ou plusieurs groupements alkyl (C₁-C₆) linéaire ou ramifié, hydroxy, alkoxy (C₁-C₆), linéaire ou ramifié, carboxy, ou alkoxy carbonyle (C₁-C₆) linéaire ou ramifié, le terme aryle désignant un groupement phényle ou naphtyle,
   le terme hétéroaryle désignant un groupement mono ou bicyclique de 4 à 11 chaînons, saturé ou insaturé, contenant de 1 à 4 hétéroatomes choisis parmi azote, oxygène et soufre,
   étant entendu que :
   - le terme éventuellement substitué affectant les expressions aryle, et hétéroaryle signifie que ces groupements sont substitués par un ou plusieurs atomes d'halogène, ou groupements alkyle (C₁-C₆) linéaire ou ramifié, trihalogénoalkyle (C₁-C₆) linéaire ou ramifié, hydroxy, alkoxy (C₁-C₆) linéaire ou ramifié, nitro, amino (éventuellement substitué par un ou deux groupements alkyle (C₁-C₆) linéaire ou ramifié, identiques ou différents), cyano, carboxy, alkylcarbonyle (C₁-C₆) linéaire ou ramifié, ou aminocarbonyle (éventuellement substitué par un ou deux groupements alkyle (C₁-C₆) linéaire ou ramifié, identiques ou différents),
leurs énantiomères, diastéréoisomères, ainsi que leurs sels d'addition à un acide ou à une base pharmaceutiquement acceptable.

Les composés de formule (I) présentant un ou plusieurs carbones asymétriques, et obtenus sous forme de mélange, peuvent être soumis à une séparation selon des techniques classiquement utilisées, et les énantiomères et/ou diastéréoisomères obtenus font partie de l'invention au même titre que les composés de formule (I).

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, oxalique, etc...

Parmi les bases pharmaceutiquement acceptables, on peut citer à titre non limitatif l'hydroxyde de sodium, l'hydroxyde de potassium, la triéthylamine, la tertbutylamine, etc...

La présente invention concerne avantageusement les composés de formule (I) pour lesquels :
- les cycles A et B forment ensemble un groupement naphtalène,
- T représente une chaîne alkylène (C₁-C₆) linéaire ou ramifiée,
- R représente un radical R' ou un groupement OR', R' représentant un groupement alkyle (C₁-C₆) linéaire ou ramifié, ou alkényle (C₂-C₆) linéaire ou ramifié,
- G₁ représente un groupement aryle éventuellement substitué ou hétéroaryle éventuellement substitué,
- G₂ représente un groupement choisi parmi : 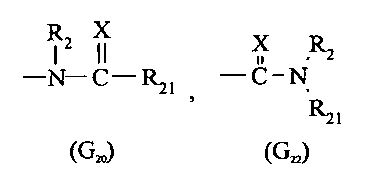
dans lesquels :
- X représente un atome d'oxygène,
- R₂ représente un atome d'hydrogène,
- R₂₁ représente un groupement alkyle (C₁-C₆) linéaire ou ramifié éventuellement substitué par un ou plusieurs atomes d'halogène, alkényle (C₂-C₆) linéaire ou ramifié, alkynyle (C₂-C₆) linéaire ou ramifié, ou cycloalkyle (C₃-C₇).

Un autre aspect avantageux de la présente invention concerne les composés de formule (I) pour lesquels :
- les cycles A et B forment ensemble un groupement tétrahydronaphtalène,
- T représente une chaîne alkylène (C₁-C₆) linéaire ou ramifiée,
- R représente un radical R' ou un groupement OR', R' représentant un groupement alkyle (C₁-C₆) linéaire ou ramifié, ou alkényle (C₂-C₆) linéaire ou ramifié,
- G₁ représente un groupement aryle éventuellement substitué ou hétéroaryle éventuellement substitué,
- G₂ représente un groupement choisi parmi : 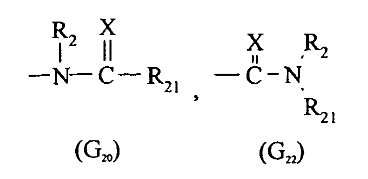
dans lesquels :
- X représente un atome d'oxygène,
- R₂ représente un atome d'hydrogène,
- R₂₁ représente un groupement alkyle (C₁-C₆) linéaire ou ramifié éventuellement substitué par un ou plusieurs atomes d'halogène, alkényle (C₂-C₆) linéaire ou ramifié, alkynyle (C₂-C₆) linéaire ou ramifié, ou cycloalkyle (C₃-C₇),

Les composés préférés de l'invention sont ceux pour lesquels T représente une chaîne alkylène de 2 ou 3 atomes de carbone.

Les groupements G₁ préférés de l'invention sont les groupements aryle (par exemple phényle) éventuellement substitué et hétéroaryle (par exemple furyle, thiényle, pyridyle) éventuellement substitué.

De façon particulière, dans les composés de l'invention, R représente un groupement R' ou OR', dans lequel R' représente un groupement alkyle (C₁-C₆) linéaire ou ramifié (par exemple un groupement méthyle), ou un groupement alkényle (C₂-C₆) linéaire ou ramifié.

De façon plus préférentielle, la présente invention concerne les composés de formule (I) pour lesquels A et B forment ensemble un groupement naphtalène, T représente une chaîne alkylène (C₁-C₆) linéaire ou ramifiée, R représente un groupement R' ou OR', R' étant un groupement alkyle (C₁-C₆) linéaire ou ramifié, ou alkényle (C₂-C₆) linéaire ou ramifié, G₁ représente un groupement aryle éventuellement substitué, ou hétéroaryle éventuellement substitué, et G₂ représente un groupement G₂₀ ou G₂₂ dans lesquels X représente un atome d'oxygène, R₂ représente un atome d'hydrogène, et R₂₁ représente un groupement choisi parmi alkyle (C₁-C₆) linéaire ou ramifié éventuellement substitué par un ou plusieurs atomes d'halogène, alkényle (C₂-C₆) linéaire ou ramifié, alkynyle (C₂-C₆) linéaire ou ramifié ou cycloalkyle (C₃-C₇).

Un autre aspect très avantageux de l'invention concerne les composés de formule (I) pour lesquels A et B forment ensemble un groupement tétrahydronaphtalène, T représente une chaîne alkylène (C₁-C₆) linéaire ou ramifiée, R représente un groupement R' ou OR', R' étant un groupement alkyle (C₁-C₆) linéaire ou ramifié, ou alkényle (C₂-C₆) linéaire ou ramifié, G₁ représente un groupement aryle éventuellement substitué, ou hétéroaryle éventuellement substitué, et G₂ représente un groupement choisi parmi G₂₀ ou G₂₂ dans lesquels X représente un atome d'oxygène, R₂ représente un atome d'hydrogène, et R₂₁ représente un groupement choisi parmi alkyle (C₁-C₆) linéaire ou ramifié éventuellement substitué par un ou plusieurs atomes d'halogène, alkényle (C₂-C₆) linéaire ou ramifié, alkynyle (C₂-C₆) linéaire ou ramifié ou cycloalkyle (C₃-C₇).

Parmi les composés préférés de l'invention on peut citer tout particulièrement les composés suivants :
- le N-[2-(7-méthoxy-3-phényl-1-naphtyl)éthyl]acétamide
- le N-{2-[3-(2-furyl)-7-méthoxy-1-naphtyl]éthyl}acétamide
- le N-{2-[7-méthoxy-3-(4-pyridyl)-1-naphtyl]éthyl}acétamide
- le N-{2-[7-méthoxy-3-(3-trifluorométhylphényl)-1-naphtyl]éthyl}acétamide
- le N-{2-[7-méthoxy-3-(3-aminophényl)-1-naphtyl]éthyl}acétamide
- le N-[2-(7-méthoxy-3-phényl-1,2,3,4-tétrahydro-1-naphtyl)éthyl]acétamide
- le N-{2-[7-méthoxy-3-(3-trifluorométhylphényl)-1,2,3,4-tétrahydro-1-naphtyl]éthyl} acétamide.

L'invention s'étend également au procédé de préparation des composés de formule (I) caractérisé en ce que l'on utilise comme produit de départ un composé de formule (II/a) : 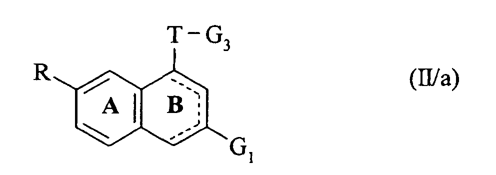 dans laquelle T, A, B, R et G₁ sont tels que définis dans la formule (I) et G₃ représente un groupement -COOH ou -NH-R₂, R₂ étant tel que défini dans la formule (I),
que l'on fait réagir, dans le cas où G₃ représente un groupement -NH-R₂,
a) avec un chlorure d'acyle de formule (III) : 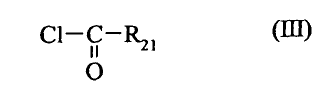
dans laquelle R₂₁ est tel que défini dans la formule (I),
ou avec l'anhydride d'acide (mixte ou symétrique) correspondant,
pour conduire à un composé de formule (I/a) : 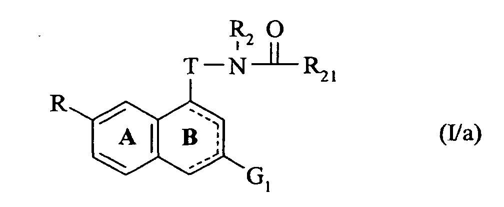 cas particulier des composés de formule (I) pour lequel T, A, B, R, R₂, R₂₁ et G₁ sont tels que définis dans la formule (I),
ou bien,
dans le cas où G₃ représente un groupement carboxy, que l'on fait réagir avec un composé de formule (V) : 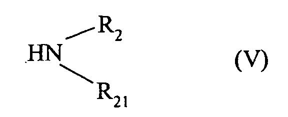 dans laquelle R₂ et R₂₁ sont tels que définis dans la formule (I),
pour conduire à un composé de formule (I/d) : 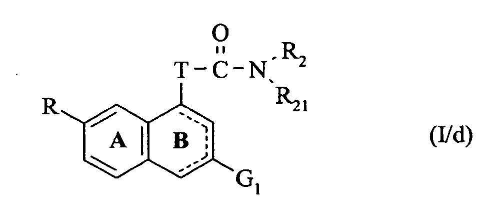 cas particulier des composés de formule (I) pour lequel T, A, B, R, R₂, R₂₁ et G₁ sont tels que définis précédemment,
composés (I/a) et (I/d) formant la totalité des composés de formule (I) :
- qui peuvent être, le cas échéant, purifiés selon une technique classique de purification,
- dont on sépare éventuellement les isomères selon une technique classique de séparation,
- que l'on transforme, si on le souhaite, en leurs sels d'addition à un acide ou à une base pharmaceutiquement acceptable.

L'invention concerne également le procédé de préparation des composés de formule (I) caractérisé en ce que l'on utilise comme produit de départ un composé de formule (II/b) : 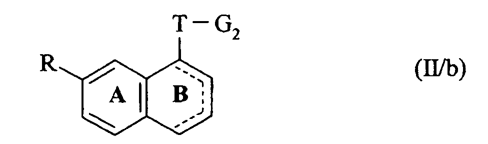 dans laquelle T, A, B, R et G₂ sont tels que définis dans la formule (I),
qui subit une réaction d'acylation en utilisant comme réactif un chlorure d'acyle Cl-CO-G₁, G₁ étant tel que défini dans la formule (I), pour conduire à un composé de formule (II/c) : 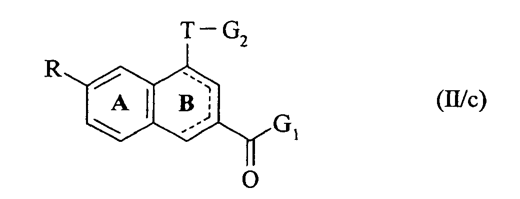 dans laquelle T, A, B, R, G₁ et G₂ sont tels que définis dans la formule (I),
qui peut être soumis à une réaction de Baeyer-Villiger pour conduire à un composé de formule (I/g) : 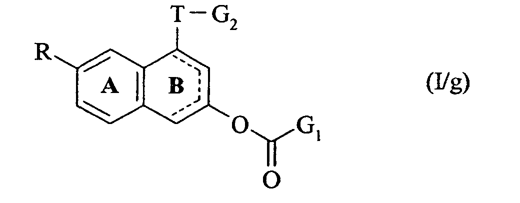 dans laquelle T, A, B, R, G₁ et G₂ ont la même signification que précédemment,
qui conduit par une réaction de saponification à un composé de formule (VI) : 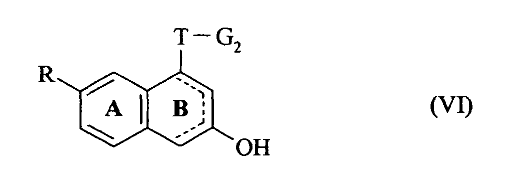 dans laquelle T, A, B, R et G₂ ont la même signification que dans la formule (I),
composé de formule (VI), dont la fonction hydroxyle est transformée en trifluorométhane sulfonate, en utilisant par exemple le phényl bis (trifluorométhane-sulfonimide) en milieu basique, pour conduire au composé de formule (VII) : 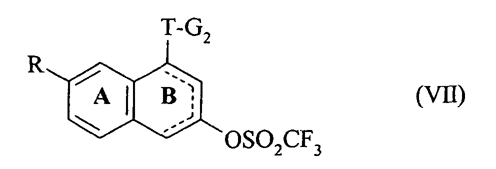 dans laquelle T, A, B, R et G₂ sont tels que définis précédemment,
que l'on peut transformer, par l'intermédiaire d'une réaction catalysée par un dérivé du palladium (0) approprié en utilisant comme réactif un dérivé de l'acide borique (G₁B(OH)₂) ou un dérivé de l'étain (G₁SnBu₃), pour lesquels G₁ est tel que défini dans la formule (I), en composé de formule (I) : 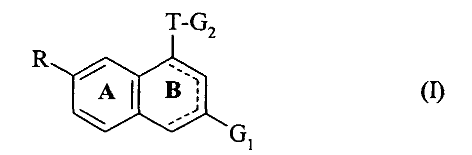 dans laquelle T, A, B, R, G₁ et G₂ ont la même signification que précédemment,
composés de formule (I),
- que l'on purifie éventuellement selon une technique classique de purification,
- dont on sépare éventuellement les isomères selon une technique classique de séparation,
- et que l'on transforme, le cas échéant, en leurs sels d'addition à un acide ou à une base pharmaceutiquement acceptable,
composés de formule (I), qui, lorsque R représente un groupement O-Alk (-Alk représentant un groupement alkyle (C₁-C₆) linéaire ou ramifié) peuvent être, lorsque cela est compatible avec les substituants présents sur la molécule, traités par le tribromure de bore pour conduire au composé hydroxylé correspondant de formule (VIII) : 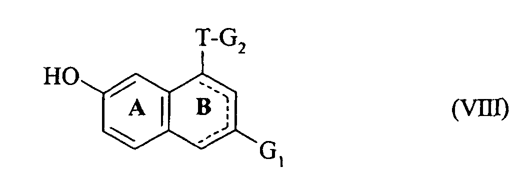 dans laquelle T, A, B, G₁ et G₂ sont tels que définis dans la formule (I),
composé (VIII) dont la fonction hydroxyle peut être :
- soit transformée en trifluorométhanesulfonate pour conduire à un composé de formule (IX) : 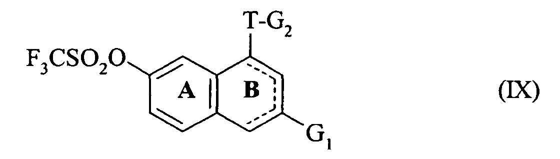 cas particulier des composés de formule (I) pour lequel T, A, B, G₁ et G₂ sont tels que définis précédemment,
   qui par l'intermédiaire d'une réaction catalysée par un dérivé du palladium (0) en utilisant comme réactif un dérivé de l'acide borique (R'B(OH)₂) ou un dérivé de l'étain (R'SnBu₃), pour lesquels R' est tel que défini dans la formule (I), permet d'obtenir un composé de formule (I/i) : 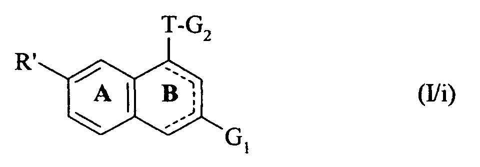 cas particulier des composés de formule (I) pour lequel T, A, B, R', G₁ et G₂ sont tels que définis dans la formule (I),
- soit soumise à une réaction de O-substitution, en milieu basique, en utilisant comme réactif le dérivé halogéné approprié, pour conduire à un composé de formule (I/j) : 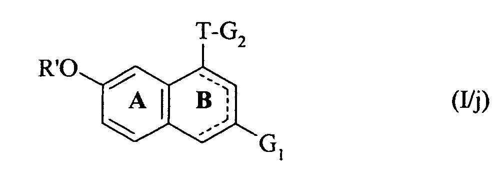 cas particulier des composés de formule (I) pour lequel T, A, B, R', G₁ et G₂ sont tels que définis précédemment,
composé de formule (I/i) et (I/j) :
- que l'on purifie éventuellement selon une technique classique de purification,
- dont on sépare éventuellement les isomères selon une technique classique de séparation,
- et que l'on transforme, le cas échéant, en leurs sels d'addition à un acide ou à une base pharmaceutiquement acceptable.

Un groupement G₁ tel que défini dans la formule (I) peut être transformé, lorsque cela est utile dans le but de simplifier le procédé ci-dessus, en un autre groupement représenté dans la description de G₁ dans la formule (I), en utilisant les réactions classiques de la chimie organique.

Les composés de formule (I/a) à (I/j) et (II) à (IX) tels que décrits dans le procédé ci-dessus, dont les cycles A et B forment ensemble un groupement naphtalène, peuvent être soumis à une réaction de réduction, lorsque cela est compatible avec les substituants présents sur la molécule de façon à conduire aux composés de formule (I) pour lesquels les cycles A et B forment ensemble un groupement choisi parmi dihydronaphtalène et tétrahydronaphtalène.

Inversement, l'aromatisation des composés de formule (I/a) à (I/j) et (II) à (IX) tels que décrits précédemment, pour lesquels les cycles A et B forment ensemble un groupement dihydronaphtalène ou tétrahydronaphtalène, lorsque cela est compatible avec les substituants présents sur la molécule, conduit aux analogues naphtaléniques de formule (I).

Les matières premières utilisées dans le procédé précédemment décrit sont soit commerciales, soit aisément accessibles à l'homme de métier selon les procédés bien connus dans la littérature.

Les composés de l'invention et les compositions pharmaceutiques les contenant s'avèrent être utiles pour le traitement des troubles du système mélatoninergique.

L'étude pharmacologique des dérivés de l'invention a en effet montré qu'ils étaient atoxiques, doués d'une très haute affinité sélective pour les récepteurs de la mélatonine et possédaient d'importantes activités sur le système nerveux central et, en particulier, on a relevé des propriétés thérapeutiques sur les troubles du sommeil, des propriétés anxiolytiques, antipsychotiques, analgésiques ainsi que sur la microcirculation qui permettent d'établir que les produits de l'invention sont utiles dans le traitement du stress, des troubles du sommeil, de l'anxiété, des dépressions saisonnières, des pathologies cardiovasculaires, des insomnies et fatigues dues aux décalages horaires, de la schizophrénie, des attaques de panique, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que les produits de l'invention possèdent des propriétés d'inhibiteurs de l'ovulation, d'immunomodulateurs et qu'ils sont susceptibles d'être utilisés dans le traitement des cancers.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonnières et des troubles du sommeil.

La présente invention a également pour objet les compositions pharmaceutiques contenant au moins un composé de formule (I) seul ou en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per.
ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en 1 ou plusieurs prises.

Les exemples suivants illustrent l'invention, mais ne la limitent en aucune façon. Les structures des composés décrits ont été confirmées par des techniques spectroscopiques usuelles.

### Exemple 1 : N-[2-(7-Méthoxy-3-trifluorométhanesulfonyloxy-1-naphtyl)éthyl] acétamide

### Stade a : N-[2-(3-Acétyl-7-méthoxy-1-naphtyl)éthyl]acétamide

A une solution de 0,16 mol (40 g) de N-[2-(7-méthoxy-1-napthyl)éthyl] acétamide (décrit dans le brevet EP 447 285) dans 350 ml de dichlorométhane, sont ajoutées 0,45 mol (60 g) de chlorure d'aluminium, à 0 °C. Puis 0,21 mol (15,3 ml) de chlorure d'acétyle sont ajoutées goutte à goutte à 0 °C. Le milieu réactionnel est agité à température ambiante pendant 1 heure, puis versé sur de la glace. La phase organique est décantée, et concentrée pour conduire au composé attendu.

### Stade b : N-[2-(3-Hydroxy-7-méthoxy-1-naphtyl)éthyl]acétamide.

A une suspension de 0,16 mol (46 g) du composé décrit au stade précédent dans 1,4 litre de dichlorométhane, sont ajoutées 0,32 mol (54 g) d'acide métachloroperoxybenzoïque. Le mélange réactionnel est agité à température ambiante pendant 20 heures. Le milieu est ensuite lavé à l'eau, et l'excès d'acide est extrait avec une solution molaire d'hydrogénocarbonate de sodium. La phase organique est décantée, séchée et concentrée. Le résidu obtenu est mis en solution dans 400 ml d'éthanol, puis 500 ml d'une solution aqueuse de soude 1M sont ajoutés. Après une heure d'agitation à température ambiante, l'éthanol est évaporé et la phase aqueuse est extraite par 500 ml de dichlorométhane. La phase aqueuse est ensuite acidifiée à pH = 1, puis extraite par 800 ml d'acétate d'éthyle. La phase organique est concentrée et purifiée par chromatographie sur gel de silice, en utilisant un mélange dichlorométhane/méthanol, 95/5, comme éluant, pour conduire au produit attendu.

### Stade c : N-[2-(7-Méthoxy-3-trifluorométhanesulfonyloxy-1-naphtyl)éthyl] acétamide

A une solution de 0,07 mol (18,15 g) du composé décrit au stade précédent dans 1 litre de dichlorométhane sont ajoutés 60 ml de triéthylamine. Le milieu réactionnel est porté à reflux jusqu'à solubilisation, puis 0,1 mol (35,8 g) de phényl bis(trifluorométhanesulfonimide) et 0,75 mol (10,5 g) de carbonate de potassium sont ajoutés. Après 4 heures à reflux, le milieu est lavé par 1 litre d'hydrogénocarbonate de sodium 1M puis par 1 litre d'acide chlorhydrique 1M. La phase organique est séchée, concentrée et purifiée par chromatographie sur gel de silice, en utilisant l'acétate d'éthyle comme éluant, pour conduire au produit du titre.

| Microanalyse élémentaire : | | | | |
|---|---|---|---|---|
| | C | H | N | S |
| % Calc | 49,10 | 4,12 | 3,58 | 8,19 |
| % Trouvé | 48,72 | 4,05 | 3,60 | 8,30 |

### Exemple 2 : N-[2-(7-Méthoxy-3-phényl-1-naphtyl)éthyl]acétamide

A une solution de 0,067 mol (24,5 g) du composé décrit dans l'exemple 1 dans 225 ml de diméthoxyéthane, sous atmosphère inerte sont ajoutés 0,1 mol (13,3 g) d'acide phénylborique, 1,8 g de tétrakistriphénylphosphine palladium (O) et 5,5 g de chlorure de lithium. Le milieu réactionnel est agité 10 mn puis 160 ml d'une solution molaire de carbonate de sodium et 110 ml d'éthanol absolu sont additionnés. La réaction est chauffée à 90 °C pendant 4 heures. Après refroidissement, 500 ml de carbonate de sodium 1 M sont ajoutés, et le milieu réactionnel est extrait deux fois par 500 ml de dichlorométhane. La phase organique est séchée, concentrée et purifiée par chromatographie sur gel de silice, en utilisant l'acétate d'éthyle comme éluant, pour conduire au composé attendu.

Point de fusion : 135 °C

### Exemple 3 : N-[2-(7-Méthoxy-3-phényl-1-napthyl)éthyl]propionamide

### Stade a : N-[2-(7-Méthoxy-3-trifluorométhanesulfonyloxy-1-napthyl)éthyl] propionamide

Le produit attendu est obtenu selon le procédé décrit dans l'exemple 1, stades a et b, à partir du N-[2-(7-méthoxy-1-naphtyl)éthyl]propionamide décrit dans le brevet EP 447 285.

### Stade b : N-[2-(7-Méthoxy-3-phényl-1-napthyl)éthyl]propionamide

Le produit attendu est obtenu selon le procédé décrit dans l'exemple 2 à partir du composé décrit au stade précédent.

### Exemple 5 : N-[2-(7-Méthoxy-3-phényl-1-napthyl)éthyl]cyclopropane carboxamide

Le produit attendu est obtenu selon le procédé décrit dans l'exemple 3 à partir du N-[2-(7-méthoxy-1-napthyl)éthyl]cyclopropane carboxamide décrit dans le brevet EP 447 285.

### Exemple 7 : N-[2-(7-Méthoxy-3-phényl-1-napthyl)éthyl]cyclobutane carboxamide

Le produit attendu est obtenu selon le procédé décrit dans l'exemple 3 à partir du N-[2-(7-méthoxy-1-naphtyl)éthyl]cyclobutane carboxamide décrit dans le brevet EP 447 285.

### Exemple 9 : N-[2-(7-Méthoxy-3-phényl-1-naphtyl)éthyl]butyramide

Le produit attendu est obtenu selon le procédé décrit dans l'exemple 3 à partir du N-[2-(7-méthoxy-1-naphtyl)éthyl]butyramide décrit dans le brevet EP 447 285.

Les composés des exemples 11 à 27 sont obtenus selon le procédé décrit dans l'exemple 2 en utilisant comme réactif un dérivé de l'acide borique ou un dérivé de l'étain approprié.

### Exemple 11 : N-{2-[3-(3-Iodophényl)-7-méthoxy-1-naphtyl]éthyl}acétamide

Point de fusion : 146 °C

| Microanalyse élémentaire : | | | |
|---|---|---|---|
| | C | H | N |
| % Calc | 56,64 | 4,53 | 3,15 |
| % Trouvé | 56,79 | 4,69 | 3,30 |

### Exemple 12 : N-{2-[7-Méthoxy-3-(4-méthoxyphényl)-1-naphtyl]éthyl}acétamide

Point de fusion : 162°C

| Microanalyse élémentaire : | | | |
|---|---|---|---|
| | C | H | N |
| % Calc | 75,62 | 6,63 | 4,01 |
| % Trouvé | 75,55 | 6,71 | 3,94 |

### Exemple 13 : N-{2-[7-Méthoxy-3-(3-méthoxyphényl)-1-naphtyl]éthyl}acétamide

Point de fusion : 109 ° C

| Microanalyse élémentaire : | | | |
|---|---|---|---|
| | C | H | N |
| % Calc | 75,62 | 6,63 | 4,01 |
| % Trouvé | 75,58 | 6,76 | 4,03 |

### Exemple 14 : N-{2-[7-Méthoxy-3-(2-méthoxyphényl)-1-naphtyl]éthyl}acétamide

Point de fusion: 101 °C

| Microanalyse élémentaire : | | | |
|---|---|---|---|
| | C | H | N |
| % Calc | 75,62 | 6,63 | 4,01 |
| % Trouvé | 75,62 | 6,80 | 4,01 |

### Exemple 15 : N-{2-[7-Méthoxy-3-(2-trifluorométhyl)phényl-1-naphtyl]éthyl} acétamide

Point de fusion: 104°C

| Microanalyse élémentaire : | | | |
|---|---|---|---|
| | C | H | N |
| % Calc | 68,21 | 5,20 | 3,62 |
| % Trouvé | 68,22 | 5,48 | 3,62 |

### Exemple 16 : N-{2-[7-Méthoxy-3-(3-trifluorométhyl)phényl-1-naphtyl]éthyl} acétamide

Point de fusion : 132 °C

| Microanalyse élémentaire : | | | |
|---|---|---|---|
| | C | H | N |
| % Calc | 68,21 | 5,20 | 3,62 |
| % Trouvé | 68,37 | 5,30 | 3,68 |

### Exemple 17 : N-{2-[7-Méthoxy-3-(4-trifluorométhyl)phényl-1-naphtyl]éthyl} acétamide

Point de fusion : 152 °C

| Microanalyse élémentaire : | | | |
|---|---|---|---|
| | C | H | N |
| % Calc | 68,21 | 5,20 | 3,62 |
| % Trouvé | 68,23 | 5,02 | 3,59 |

### Exemple 18 : N-{2-[7-Méthoxy-3-(1-naphtyl)-1-naphtyl]éthyl}acétamide

| Microanalyse élémentaire : | | | |
|---|---|---|---|
| | C | H | N |
| % Calc | 81,27 | 6,27 | 3,71 |
| % Trouvé | 81,23 | 6,43 | 3,79 |

### Exemple 20 : N-{2-[(3-(2-Furyl)-7-méthoxy)-1-naphtyl]éthyl}acétamide

Point de fusion: 138°C

| Microanalyse élémentaire : | | | |
|---|---|---|---|
| | C | H | N |
| % Calc | 73,77 | 6,19 | 4,53 |
| % Trouvé | 73,72 | 6,23 | 4,65 |

### Exemple 21 : N-{2-[(7-Méthoxy-3-(2-thiényl))-1-naphtyl]éthyl]}acétamide

Point de fusion : 111 °C

| Microanalyse élémentaire : | | | | |
|---|---|---|---|---|
| | C | H | N | S |
| % Calc | 70,13 | 5,88 | 4,30 | 9,85 |
| % Trouvé | 70,05 | 5,92 | 4,30 | 9,73 |

### Exemple 22 : N-[2-(7-Méthoxy-3-(4-nitrophenyl)-1-naphtyl)éthyl]acétamide

### Exemple 23 : N-[2-(7-Méthoxy-3-(3-nitrophényl)-1-napthyl)éthyl]acétamide

Point de fusion : 143-144 °C

| Microanalyse élémentaire : | | | |
|---|---|---|---|
| | C | H | N |
| % Calc | 69,22 | 5,53 | 7,69 |
| % Trouvé | 69,32 | 5,74 | 7,25 |

### Exemple 24 : N-[2-(7-Méthoxy-3-(2-nitrophényl)-1-naphtyl)éthyl]acétamide

### Exemple 25 : N-[2-(3-(4-Aminophényl)-7-méthoxy-1-naphtyl)éthyl]acétamide, chlorhydrate

### Exemple 26 : N-[2-(3-(3-Aminophényl)-7-méthoxy-1-naphtyl)éthyl]acétamide chlorhydrate

Point de fusion: 209-210 °C

| Microanalyse élémentaire : | | | | |
|---|---|---|---|---|
| | C | H | N | Cl |
| % Calc | 68,01 | 6,25 | 7,55 | 9,56 |
| % Trouvé | 69,19 | 6,23 | 7,55 | 9,45 |

### Exemple 27: N-[2-(3-(2-Aminophényl)-7-méthoxy-1-napthyl)éthyl]acétamide, Chlorhydrate

### Exemple 47 : N-[2-(3-Phényl-7-vinyl-1-naphtyl)éthyl]acétamide

Le produit attendu est obtenu selon le procédé décrit dans l'exemple 46 en remplaçant l'acide formique par le tributylvinylétain.

Point de fusion : 132°C

| Microanalyse élémentaire : | | | |
|---|---|---|---|
| | C | H | N |
| % Calc | 83,78 | 6,71 | 4,44 |
| % Trouvé | 82,97 | 6,82 | 4,45 |

### Exemple 48 : N-[2-(7-Ethyl-3-phényl-1-naphtyl)éthyl]acétamide

Une solution de 0,02 mol (7 g) du composé décrit dans l'exemple 47 dans 400 ml d'éthanol est agité sous pression atmosphérique d'hydrogène, à température ambiante, en présence de 100 mg de palladium sur charbon, pendant 2 heures. Après filtration du catalyseur et évaporation du solvant, le résidu est purifié par chromatographie sur gel de silice pour conduire au composé attendu.

Point de fusion : 130°C

| Microanalyse élémentaire | | | |
|---|---|---|---|
| | C | H | N |
| % Calc | 83,24 | 7,30 | 4,41 |
| % Trouvé | 83,08 | 7,55 | 4,47* |

### Exemple 49 : N-[2-(3-Phényl-7-propyloxy-1-naphtyl)éthyl]acétamide

A une solution de 0,023 mol (7 g) de N-[2-(7-Hydroxy-3-phenyl-1-naphtyl)éthyl]acétamide 44 dans 250 ml d'acétone, sont ajoutées 0,046 mol (6,3 g) de carbonate de potassium. Le milieu réactionnel est porté à reflux pendant 15 minutes, puis 0,046 mol (4,5 ml) d'iodopropane sont ajoutées. La réaction est chauffée à reflux pendant 18 heures. Après refroidissement et filtration, le filtrat est concentré. Le résidu est repris dans l'acétate d'éthyle, extrait, et la phase organique est lavée par une solution de soude à 20 %, séchée et concentrée pour conduire au composé attendu.

Les composés des exemples 50 à 52 sont obtenus selon le procédé décrit dans l'exemple 49 en utilisant le dérivé halogéné approprié.

### Exemple 50 : N-[2-(7-Butyloxy-3-phényl-1-naphtyl)éthyl]acétamide

### Exemple 51 : N-[2-(7-Hexyloxy-3-phényl-1-naphtyl)éthyl]acétamide

### Exemple 69 : N-Méthyl-4-(7-méthoxy-3-trifluorométhanesulfonyloxy-1-naphtyl) butanamide

Le produit attendu est obtenu selon le procédé décrit dans l'exemple 1 en utilisant comme produit de départ la N-méthyl-4-(7-méthoxy-1-napthyl)butanamide (décrit dans la demande EP 745 584).

### Exemple 70 : N-Méthyl-4-[3-(2-furyl)-7-méthoxy-1-naphtyl]butanamide

Le produit attendu est obtenu selon le procédé décrit dans l'exemple 21 en utilisant comme produit de départ le composé décrit dans l'exemple 69, et comme réactif le tributyl(2-furyl)étain.

Selon les procédés semblables à ceux décrits pour les exemples 69 et 70, les composés suivants peuvent être obtenus.

### Exemple 71 : N-Propyl-4-[3-(2-furyl)-7-méthoxy-1-naphtyl]butanamide

### Exemple 72 : N-Méthyl-4-[7-méthoxy-3-(2-pyridyl)-1-naphtyl]butanamide

Les composés des exemples 73 à 100 sont obtenus par réduction sous pression d'hydrogène et à une température de 100 à 120 °C, des dérivés naphtaléniques décrits dans les exemples précédents, en utilisant le catalyseur approprié.

### Exemple 73 : N-[2-(7-Méthoxy-3-phényl-1,2,3,4-tétrahydro-1-naphtyl)éthyl] acétamide

Point de fusion : 105-107 °C

### Exemple 74 : N-[2-(7-Méthoxy-3-phényl-1,2,3,4-tétrahydro-1-naphtyl)éthyl] cyclopropanecarboxamide

### Exemple 76 : N-[2-(7-Méthoxy-3-phényl-1,2,3,4-tétrahydro-1-naphtyl)éthyl] butyramide

### Exemple 78 : N-{2-[3-(2-Furyl)-7-méthoxy-1,2,3,4-tétrahydro-1-naphtyl]éthyl} acétamide

### Exemple 79 : N-{2-[7-Méthoxy-3-(2-thiényl)-1,2,3,4-tétrahydro-1-naphtyl]éthyl} acétamide

### Exemple 80 : N-[2-(7-Ethyl-3-phényl-1,2,3,4-tétrahydro-1-naphtyl)éthyl]acétamide

### Exemple 81 : N-[2-(7-Méthoxy-3-(4-méthoxyphényl)-1,2,3,4-tétrahydro-1-naphtyl) éthyl]acétamide

### Exemple 82 : N-[2-(7-Méthoxy-3-(3-méthoxyphényl)-1,2,3,4-tétrahydro-1-naphtyl) éthyl]acétamide

### Exemple 83 : N-[2-(7-Méthoxy-3-(3-trifluorométhylphényl)-1,2,3,4-tétrahydro-1-naphtyl)éthyl]acétamide

### Exemple 84 : N-[2-(3-(4-Aminophényl)-7-méthoxy-1,2,3,4-tétrahydro-1-naphtyl) éthyl]acétamide, chlorhydrate

### Exemple 85 : N-[2-(3-(3-Aminophényl)-7-méthoxy-1,2,3,4-tétrahydro-1-naphtyl) éthyl]acétamide, chlorhydrate

### Exemple 86 : N-[2-(3-(2-Aminophényl)-7-méthoxy-1,2,3,4-tétrahydro-1-naphtyl) éthyl]acétamide, chlorhydrate

### Exemple 93 : N-Méthyl-4-[3-(2-Furyl)-7-méthoxy-1,2,3,4-tétrahydro-1-naphtyl] butanamide

Les composé des exemples 95 à 99 sont obtenus selon le procédé décrit dans l'exemple 49, en utilisant comme produit de départ le composé décrit dans l'exemple 101, et comme réactif le dérivé halogéné approprié.

### Exemple 95 : N-[2-(3-Phényl-7-vinyloxy-1,2,3,4-tétrahydro-1-naphtyl)éthyl) acétamide

### Exemple 96 : N-[2-(3-Phényl-7-propyloxy-1,2,3,4-tétrahydro-1-naphtyl)éthyl] acétamide

### Exemple 97 : N-[2-(7-Butyloxy-3-phényl-1,2,3,4-tétrahydro-1-naphtyl)éthyl] acétamide

### Exemple 98 : N-[2-(7-Hexyloxy-3-phényl-1,2,3,4-tétrahydro-1-naphtyl)éthyl] acétamide

De la même façon que pour les composés des exemples 73 à 93, les composés des exemples 100 à 106 sont obtenus.

### Exemple 100 : N-[2-(7-Méthoxy-3-phényl-1,2,3,4-tétrahydro-1-naphtyl)éthyl] cyclobutanecarboxamide

### Exemple 101 : N-{2-[(1R,3R)et(1S,3S)-7-Méthoxy-3-phényl-1,2,3,4-tétrahydro-1-naphtyl]éthyl}cyclobutanecarboxamide

Point de fusion : 141-142 °C

### Exemple 102 : N-{2-[(1R,3S)et(1S,3R)-7-Méthoxy-3-phényl-1,2,3,4-tétrahydro-1-naphtyl]éthyl}cyclobutanecarboxamide

Point de fusion : 110-111 °C

### Exemple 103 : N-{2-[(1R,3R)et(1S,3S)-7-Méthoxy-3-phényl-1,2,3,4-tétrahydro-1-naphtyl]éthyl}cyclopropanecarboxamide

Point de fusion : 145 °C

### Exemple 104 : N-{2-[(1R,3S)et(1S,3R)-7-Méthoxy-3-phényl-1,2,3,4-tétrahydro-1-naphtyl]éthyl}cyclopropanecarboxamide

### Exemple 105 : N-{2-[(1R,3R)et(1S,3S)-7-Méthoxy-3-phényl-1,2,3,4-tétrahydro-1-naphtyl]éthyl}acétamide

### Exemple 106 : N-{2-[(1R,3S)et(1S,3R)-7-Méthoxy-3-phényl-1,2,3,4-tétrahydro-1-naphtyl]éthyl}acétamide

### Exemple 107 : N-[2-(7-Ethyl-3-phényl-1-naphtyl)éthyl]cyclobutanecarboxamide

### Stade a : 2-(7-Ethyl-3-phényl-1-naphtyl)éthylamine

A une solution de 1,6 mmol (0,5 g) du composé décrit dans l'exemple 48 dans 20 ml d'éthanol sont ajoutés 10 ml d'une solution aqueuse de soude à 20 %. Le milieu réactionnel est chauffé à reflux pendant 5 heures. Après refroidissement, et évaporation, le résidu est repris dans un mélange dichlorométhane/eau, et extrait. La phase organique est séchée sur sulfate de magnésium, et concentrée pour conduire au composé attendu.

### Stade b : N-[2-(7-Ethyl-3-phényl-1-naphtyl)éthyl]cyclobutanecarboxamide

1,6 mmol (0,42 g) du composé décrit au stade précédent sont dissoutes dans un mélange de 8 ml de dichlorométhane et 4 ml d'eau. Sous agitation vigoureuse, 4,1 mmol (0,56 g) de carbonate de potassium et 1,81 mmol (0,21 g) de chlorure de l'acide cyclobutanoïque sont ajoutées successivement, à 0°C. La réaction est agitée vigoureusement à température ambiante pendant 2 heures. Le milieu réactionnel est extrait au dichlorométhane et les phases organiques regroupées sont séchées sur sulfate de magnésium et concentrées pour conduire au composé attendu.

| Microanalyse élémentaire : | | | |
|---|---|---|---|
| | C | H | N |
| % Calc | 83,99 | 7,61 | 3,92 |
| % Trouvé | 83,44 | 7,62 | 3,99 |

### Exemple 108 : N-[2-(7-Ethyl-3-phényl-1-naphtyl)éthyl]trifluoroacétamide

Le produit attendu est obtenu selon le procédé décrit dans l'exemple 107 en utilisant, au stade b, le chlorure de l'acide trifluoroacétique à la place du chlorure de l'acide cyclobutanoïque.

| Microanalyse élémentaire : | | | |
|---|---|---|---|
| | C | H | N |
| % Calc | 71,15 | 5,43 | 3,77 |
| % Trouvé | 71,44 | 5,67 | 3,80 |

### Exemple 109 : N-[2-(7-Ethyl-3-phényl-1-naphtyl)éthyl]-3-butènamide

Le produit attendu est obtenu selon le procédé décrit dans l'exemple 107 en utilisant, au stade b, le chlorure de l'acide vinylacétique à la place du chlorure de l'acide cyclobutanoïque.

| Microanalyse élémentaire : | | | |
|---|---|---|---|
| | C | H | N |
| % Calc | 83,93 | 7,34 | 4,08 |
| % Trouvé | 84,36 | 7,21 | 4,22 |

### Exemple 112 : N-(2-{7-Méthoxy-3-[3-(trifluorométhyl)phényl]-1-naphtyl}éthyl)-2-iodoacétamide

Le produit attendu est obtenu selon le procédé décrit dans l'exemple 107, en remplaçant, au stade a, le composé de l'exemple 48 par le composé de l'exemple 16, et au stade b, en remplaçant le chlorure de l'acide cyclobutanoïque par le chlorure de l'acide iodoacétique.

| Microanalyse élémentaire : | | | | |
|---|---|---|---|---|
| | C | H | N | I |
| % Calc | 51,48 | 3,73 | 2,73 | 24,72 |
| % Trouvé | 51,83 | 3,82 | 2,79 | 24,96 |

Les composés des exemples 113 à 115 sont obtenus selon le procédé décrit dans l'exemple 2 en utilisant comme réactif le dérivé de l'acide borique ou de l'étain approprié.

### Exemple 113 : N-{2-[7-Méthoxy-3-(4-pyridyl)-1-naphtyl]éthyl}acétamide, chlorhydrate

| Microanalyse élémentaire : | | | |
|---|---|---|---|
| | C | H | N |
| % Calc | 67,32 | 5,93 | 7,85 |
| % Trouvé | 67,27 | 5,96 | 7,64 |

### Exemple 114 : N-{2-[7-Méthoxy-3-(3-pyridyl)-1-naphtyl]éthyl}acétamide

Point de fusion : 111-113 °C

| Microanalyse élémentaire : | | | |
|---|---|---|---|
| | C | H | N |
| % Calc | 74,98 | 6,29 | 8,74 |
| % Trouvé | 75,05 | 6,55 | 8,81 |

### Exemple 115 : N-{2-[7-Méthoxy-3-(2-pyridyl)-1-naphtyl]éthyl}acétamide

Point de fusion : 150-152 °C

| Microanalyse élémentaire : | | | |
|---|---|---|---|
| | C | H | N |
| % Calc | 74,98 | 6,29 | 8,74 |
| % Trouvé | 75,05 | 6,55 | 8,81 |

### ETUDE PHARMACOLOGIQUE

### EXEMPLE A : Etude de la toxicité aiguë

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50, entraînant la mort de 50 % des animaux, a été évaluée et a montré la faible toxicité des composés de l'invention.

### EXEMPLE B : Etude de liaison aux récepteurs de la mélatonine sur des cellules de la pars tuberalis de mouton

Les études de liaison aux récepteurs de la mélatonine des composés de l'invention ont été réalisées selon les techniques classiques sur les cellules de la pars tuberalis de mouton. La pars tuberalis de l'adénohypophyse est en effet caractérisée, chez les mammifères, par une haute densité en récepteurs de la mélatonine (Journal of Neuroendocrinology, 1, pp 1-4, 1989).

### Protocole

1) Les membranes de pars tuberalis de mouton sont préparées et utilisées comme tissu cible dans des expériences de saturation pour déterminer les capacités et affinités de liaison pour la 2-[¹²⁵I] - iodomélatonine.
2) Les membranes de pars tuberalis de mouton sont utilisées comme tissu cible, avec les différents composés à tester, dans des expériences de liaison compétitive par rapport à la mélatonine.

Chaque expérience est réalisée en triple et une gamme de concentrations différentes est testée pour chaque composé. Les résultats permettent de déterminer, après traitement statistique, les affinités de liaison du composé testé.

### Résultats

Il apparaît que les composés de l'invention possèdent une puissante affinité pour les récepteurs de la mélatonine.

### EXEMPLE C : Etude de liaison aux récepteurs MEL₁ₐ et MEL_{1b} de la mélatonine

Les expériences de liaison aux récepteurs MEL₁ₐ ou MEL_{1b} sont réalisées en utilisant la 2-[¹²⁵I]-mélatonine comme radioligand de référence. La radioactivité retenue est déterminée à l'aide d'un compteur à scintillation liquide Beckman® LS 6000.
Des expériences de liaison compétitive sont ensuite réalisées en triple, avec les différents composés à tester. Une gamme de concentrations différentes est testée pour chaque composé. Les résultats permettent de déterminer les affinités de liaison des composés testés (IC₅₀).

Ainsi, les valeurs d'IC₅₀ trouvées pour les composés de l'invention montrent que la liaison des composés testés est très puissante pour l'un ou l'autre des sous-types de récepteurs MEL₁ₐ ou MEL_{1b}, ces valeurs se situant dans un intervalle de 0,1 à 10nM.

### EXEMPLE D : Test des quatre plaques

Les produits de l'invention sont administrés par voie oesophagienne à des lots de dix souris. Un lot reçoit du sirop de gomme. Trente minutes après l'administration des produits à étudier, les animaux sont placés dans des habitacles dont le plancher comprend quatre plaques métalliques. Chaque fois que l'animal passe d'une plaque à l'autre, il reçoit une légère décharge électrique (0,35 mA). Le nombre de passages est enregistré pendant une minute. Après administration, les composés de l'invention augmentent de façon significative le nombre de passages ce qui montre l'activité anxiolytique des dérivés de l'invention.

### EXEMPLE E : Action des composés de l'invention sur les rythmes circadiens d'activité locomotrice du rat

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et, en particulier, sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### Protocole expérimental

Des rats mâles Long Evans âgés de un mois sont soumis dès leur arrivée au laboratoire à un cycle lumineux de 12h de lumière par 24h (LD 12 : 12).
Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité par le rythme lumineux,
- disparition de l'entraînement des rythmes en obscurité permanente,
- entraînement par l'administration quotidienne de la molécule ; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### Résultats :

Il apparaît clairement que les composés de l'invention permettent d'agir de façon puissante sur le rythme circadien *via* le système mélatoninergique.

### EXEMPLE F : Activité Antiarythmique

### Protocole

### (Ref : LAWSON J.W. et al. J. Pharmacol. Expert. Therap., 1968, 160, pp 22-31)

La substance testée est administrée par voie intrapéritonéale à un groupe de 3 souris 30 min avant l'exposition à une anesthésie par le chloroforme. Les animaux sont ensuite observés pendant 15 min. L'absence d'enregistrement d'arythmies et de fréquences cardiaques supérieures à 200 battements / min (témoin : 400-480 battements / min) chez deux animaux au moins indique une protection significative.

### EXEMPLE G : Composition pharmaceutique : Comprimés

| | |
|---|---|
| 1000 comprimés dosés à 5 mg du composé de l'exemple 2 | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) 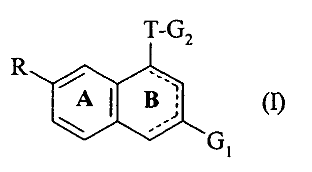 dans laquelle :
• les cycles A et B forment ensemble un groupement naphtalène, 2,3-dihydronaphtalène, 1,4-dihydronaphtalène, 1,2-dihydronaphtalène, ou tétrahydronaphtalène,
• T représente une chaîne alkylène (C₁-C₆) linéaire ou ramifiée éventuellement substituée par un ou plusieurs groupements alkyle (C₁-C₆) linéaire ou ramifié, hydroxy, alkoxy (C₁-C₆) linéaire ou ramifié, carboxy, ou alkoxycarbonyle (C₁-C₆) linéaire ou ramifié,
• R représente un radical R' ou un groupement OR', R' représentant un groupement alkyle (C₁-C₆) linéaire ou ramifié, ou alkényle (C₂-C₆) linéaire ou ramifié,
• G₁ représente un groupement aryle éventuellement substitué ou hétéroaryle éventuellement substitué,
• G₂ représente un groupement choisi parmi : 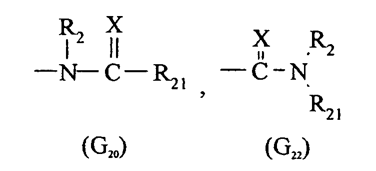
dans lesquels :
- X représente un atome d'oxygène,
- R₂ représente un atome d'hydrogène,
- R₂₁ représente un groupement alkyle (C₁-C₆) linéaire ou ramifié éventuellement substitué par un ou plusieurs atomes d'halogène, alkényle (C₂-C₆) linéaire ou ramifié, alkynyle (C₂-C₆) linéaire ou ramifié, ou cycloalkyle (C₃-C₇),
étant entendu que :
- lorsque G₂ représente un groupement G₂₂, alors G₁ représente un groupement hétéroaryle (différent d'un pyridyle) éventuellement substitué,
- lorsque G₂ représente un groupement G₂₀ et G₁ est différent d'un groupement hétéroaryle éventuellement substitué, alors T représente une chaîne méthylène ou éthylène éventuellement substituée par un ou plusieurs groupement alkyl (C₁-C₆) linéaire ou ramifié, hydroxy, alkoxy (C₁-C₆) linéaire ou ramifié, carboxy, ou alkoxycarbonyl (C₁-C₆) linéaire ou ramifié, le terme aryle désignant un groupement phényle ou naphtyle,
le terme hétéroaryle désignant un groupement mono ou bicyclique de 4 à 11 chaînons, saturé ou insaturé, contenant de 1 à 4 hétéroatomes choisis parmi azote, oxygène et soufre,
étant entendu que :
- le terme éventuellement substitué affectant les expressions aryle et hétéroaryle signifie que ces groupements sont substitués par un ou plusieurs atomes d'halogène, ou groupements alkyle (C₁-C₆) linéaire ou ramifié, trihalogénoalkyle (C₁-C₆) linéaire ou ramifié, hydroxy, alkoxy (C₁-C₆) linéaire ou ramifié, nitro, amino (éventuellement substitué par un ou deux groupements alkyle (C₁-C₆) linéaire ou ramifié, identiques ou différents), cyano, carboxy, alkylcarbonyle (C₁-C₆) linéaire ou ramifié, ou aminocarbonyle (éventuellement substitué par un ou deux groupements alkyle (C₁-C₆) linéaire ou ramifié, identiques ou différents),
leurs énantiomères, diastéréoisomères, ainsi que leurs sels d'addition à un acide ou à une base pharmaceutiquement acceptable.

2. Composés de formule (I) selon la revendication 1 pour lesquels :
• les cycles A et B forment ensemble un groupement naphtalène,
• représente une chaîne alkylène (C₁-C₆) linéaire ou ramifiée,
• R représente un radical R' ou un groupement OR', R' représentant un groupement alkyle (C₁-C₆) linéaire ou ramifié, ou alkényle (C₂-C₆) linéaire ou ramifié,
• G₁ représente un groupement aryle éventuellement substitué ou hétéroaryle éventuellement substitué,
• G₂ représente un groupement choisi parmi : 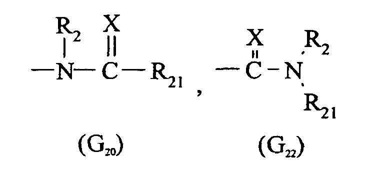
dans lesquels :
- X représente un atome d'oxygène,
- R₂ représente un atome d'hydrogène,
- R₂₁ représente un groupement alkyle (C₁-C₆) linéaire ou ramifié éventuellement substitué par un ou plusieurs atomes d'halogène, alkényle (C₂-C₆) linéaire ou ramifié, alkynyle (C₂-C₆) linéaire ou ramifié, ou cycloalkyle (C₃-C₇),
leurs énantiomères, diastéréoisomères, ainsi que leurs sels d'addition à un acide ou à une base pharmaceutiquement acceptable.

3. Composés de formule (I) selon la revendication 1 pour lesquels :
• les cycles A et B forment ensemble un groupement tétrahydronaphtalène,
• T représente une chaîne alkylène (C₁-C₆) linéaire ou ramifiée,
• R représente un radical R' ou un groupement OR', R' représentant un groupement alkyle (C₁-C₆) linéaire ou ramifié, ou alkényle (C₂-C₆) linéaire ou ramifié,
• G₁ représente un groupement aryle éventuellement substitué ou hétéroaryle éventuellement substitué,
• G₂, représente un groupement choisi parmi : 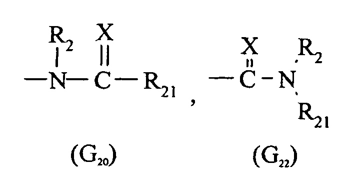
dans lesquels :
- X représente un atome d'oxygène,
- R₂ représente un atome d'hydrogène,
- R₂₁ représente un groupement alkyle (C₁-C₆) linéaire ou ramifié éventuellement substitué par un ou plusieurs atomes d'halogène, alkényle (C₂-C₆) linéaire ou ramifié, alkynyle (C₂-C₆) linéaire ou ramifié, ou cycloalkyle (C₃-C₇),
leurs énantiomères, diastéréoisomères, ainsi que leurs sels d'addition à un acide ou à une base pharmaceutiquement acceptable.

4. Composés de formule (I) selon la revendication 1 pour lesquels T représente une chaîne alkylène de 2 ou 3 atomes de carbone, leurs énantiomères, diastéréoisomères, ainsi que leurs sels d'addition à un acide ou à une base pharmaceutiquement acceptable.

5. Composés de formule (I) selon la revendication 1 pour lesquels G₁ représente un groupement aryle éventuellement substitué, leurs énantiomères, diastéréoisomères, ainsi que leurs sels d'addition à un acide ou à une base pharmaceutiquement acceptable.

6. Composés de formule (I) selon la revendication 1 pour lesquels G₁ représente un groupement hétéroaryle éventuellement substitué, leurs énantiomères, diastéréoisomères, ainsi que leurs sels d'addition à un acide ou à une base pharmaceutiquement acceptable.

7. Composés de formule (I) selon la revendication 1 pour lesquels R représente un groupement R', R' étant un groupement alkyle (C₁-C₆) linéaire ou ramifié ou alkényle (C₂-C₆) linéaire ou ramifié, leurs énantiomères, diastéréoisomères, ainsi que leurs sels d'addition à un acide ou à une base pharmaceutiquement acceptable.

8. Composés de formule (I) selon la revendication 1 pour lesquels R représente un groupement OR', R' étant un groupement alkyle (C₁-C₆) linéaire ou ramifié, ou alkényle (C₂-C₆) linéaire ou ramifié, leurs énantiomères, diastéréoisomères, ainsi que leurs sels d'addition à un acide ou à une base pharmaceutiquement acceptable.

9. Composé de formule (I) selon la revendication 1 qui est le N-[2-(7-méthoxy-3-phényl-1-naphtyl)éthyl]acétamide.

10. Composé de formule (I) selon la revendication 1 qui est le N-{2-[7-méthoxy-3-(3-trifluorométhylphényl)-1-naphtyl]éthyl}acétamide.

11. Composé de formule (I) selon la revendication 1 qui est le N-{2-[7-méthoxy-3-(3-aminophényl)-1-naphtyl]éthyl}acétamide, ainsi que ses sels d'addition à un acide ou à une base pharmaceutiquement acceptable.

12. Composés de formule (I) selon la revendication 1 qui sont les :
- N-{2-[3-(2-furyl)-7-méthoxy-1-naphtyl]éthyl}acétamide,
- N-{2-[7-méthoxy-3-(4-pyridyl)-1-naphtyl]éthyl}acétamide.

13. Composés de formule (I) selon la revendication 1 qui sont les :
- N-[2-(7-méthoxy-3-phényl-1,2,3,4-tétrahydro-1-naphtyl)éthyl]acétamide,
- N-{2-[7-méthoxy-3-(3-trifluorométhylphényl)-1,2,3,4-tétrahydro-1-naphtyl]éthyl} acétamide

14. Procédé de préparation des composés de formule (I) selon la revendication 1 **caractérisé en ce que** l'on utilise comme produit de départ un composé de formule (II/a) : 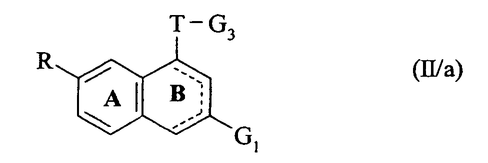 dans laquelle T, A, B, R et G₁ sont tels que définis dans la formule (I) et G₃ représente un groupement -COOH ou -NH-R₂, R₂ étant tel que défini dans la formule (I),
que l'on fait réagir, dans le cas où G₃ représente un groupement -NH-R₂,
a) avec un chlorure d'acyle de formule (III) : 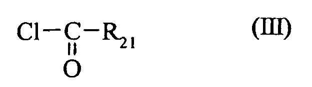
dans laquelle R₂₁ est tel que défini dans la formule (I),
ou avec l'anhydride d'acide (mixte ou symétrique) correspondant,
pour conduire à un composé de formule (I/a) : 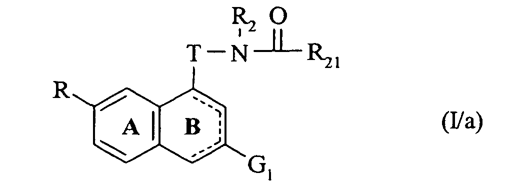 cas particulier des composés de formule (I) pour lequel T, A, B, R, R₂, R₂₁ et G₁ sont tels que définis dans la formule (I),
ou bien,
dans le cas où G₃ représente un groupement carboxy, que l'on fait réagir avec un composé de formule (V) : 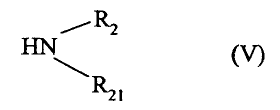 dans laquelle R₂ et R₂₁ sont tels que définis dans la formule (I),
pour conduire à un composé de formule (I/d) : 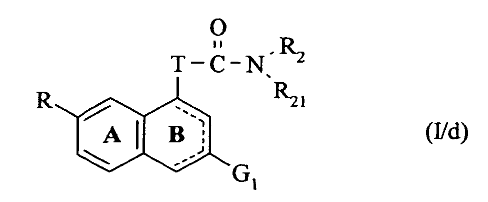 cas particulier des composés de formule (I) pour lequel T, A, B, R, R₂, R₂₁ et G₁ sont tels que définis précédemment,
composés (I/a) et (I/d) formant la totalité des composés de formule (I),
- qui peuvent être, le cas échéant, purifiés selon une technique classique de purification,
- dont on sépare éventuellement les isomères selon une technique classique de séparation,
- que l'on transforme, si on le souhaite, en leurs sels d'addition à un acide ou à une base pharmaceutiquement acceptable.

15. Procédé de préparation des composés de formule (I) selon la revendication 1 **caractérisé en ce que** l'on utilise comme produit de départ un composé de formule (II/b) : 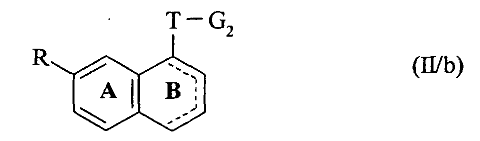 dans laquelle T, A, B, R et G₂ sont tels que définis dans la formule (I),
qui subit une réaction d'acylation en utilisant comme réactif un chlorure d'acyle Cl-CO-G₁, G₁ étant tel que défini dans la formule (I), pour conduire à un composé de formule (II/c) : 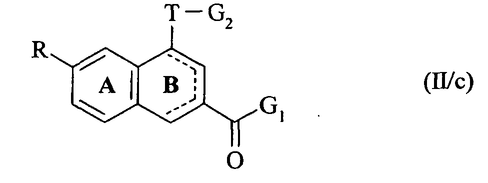 dans laquelle T, A, B, R, G₁ et G₂ sont tels que définis dans la formule (I),
qui peut être soumis à une réaction de Baeyer-Villiger pour conduire à un composé de formule (I/g) : 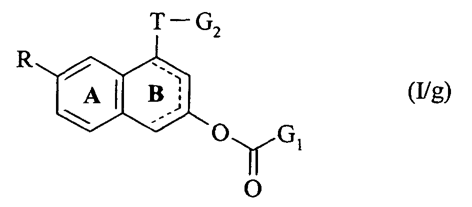 dans laquelle T, A, B, R, G₁ et G₂ ont la même signification que précédemment,
qui conduit par une réaction de saponification à un composé de formule (VI) : 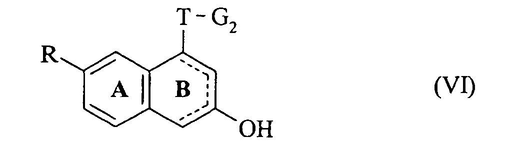 dans laquelle T, A, B, R et G₂ ont la même signification que dans la formule (I),
composé de formule (VI), dont la fonction hydroxyle est transformée en trifluorométhane sulfonate, en utilisant par exemple le phényl bis (trifluorométhane-sulfonimide) en milieu basique, pour conduire au composé de formule (VII) : 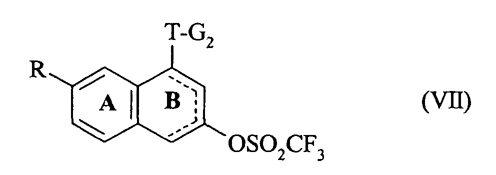 dans laquelle T, A, B, R et G₂ sont tels que définis précédemment,
que l'on peut transformer, par l'intermédiaire d'une réaction catalysée par un dérivé du palladium (0) approprié en utilisant comme réactif un dérivé de l'acide borique (G₁B(OH)₂) ou un dérivé de l'étain (G₁SnBu₃), pour lesquels G₁ est tel que défini dans la formule (I), en composé de formule (I) : 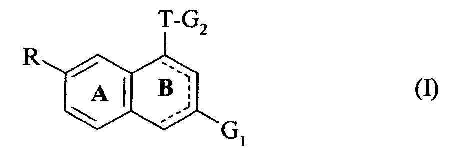 dans laquelle T, A, B, R, G₁ et G₂ ont la même signification que précédemment,
composés de formule (I),
- que l'on purifie éventuellement selon une technique classique de purification,
- dont on sépare éventuellement les isomères selon une technique classique de séparation,
- et que l'on transforme, le cas échéant, en leurs sels d'addition à un acide ou à une base pharmaceutiquement acceptable,
composés de formule (I), qui, lorsque R représente un groupement O-Alk (-Alk représentant un groupement alkyle (C₁-C₆) linéaire ou ramifié) peuvent être, lorsque cela est compatible avec les substituants présents sur la molécule, traités par le tribromure de bore pour conduire au composé hydroxylé correspondant de formule (VIII) : 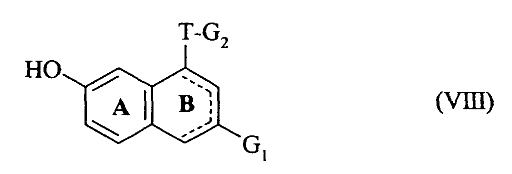 dans laquelle T, A, B, G₁ et G₂ sont tels que définis dans la formule (I),
composé (VIII) dont la fonction hydroxyle peut être :
- soit transformée en trifluorométhanesulfonate pour conduire à un composé de formule (IX) : 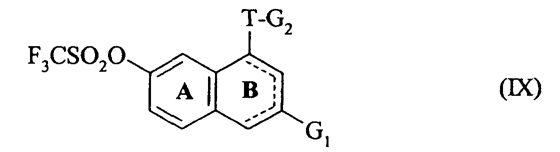 cas particulier des composés de formule (I) pour lequel T, A, B, G₁ et G₂ sont tels que définis précédemment,
qui par l'intermédiaire d'une réaction catalysée par un dérivé du palladium (0) en utilisant comme réactif un dérivé de l'acide borique (R'B(OH)₂) ou un dérivé de l'étain (R'SnBu₃), pour lesquels R' est tel que défini dans la formule (I), permet d'obtenir un composé de formule (I/i) : 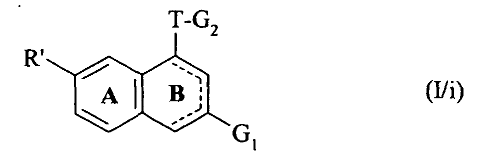 cas particulier des composés de formule (I) pour lequel T, A, B, R', G₁ et G₂ sont tels que définis dans la formule (I),
- soit soumise à une réaction de O-substitution, en mileu basique, en utilisant comme réactif le dérivé halogéné approprié, pour conduire à un composé de formule (I/j) : 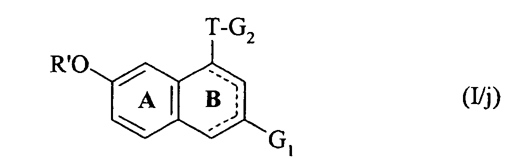 cas particulier des composés de formule (I) pour lequel T, A, B, R', G₁ et G₂ sont tels que
définis précédemment,
composé de formule (I/i) et (I/j) :
- que l'on purifie éventuellement selon une technique classique de purification,
- dont on sépare éventuellement les isomères selon une technique classique de séparation,
- et que l'on transforme, le cas échéant, en leurs sels d'addition à un acide ou à une base pharmaceutiquement acceptable.

16. Compositions pharmaceutiques contenant comme principe actif au moins un composé selon l'une quelconque des revendications 1 à 13, seul ou en combinaison avec un ou plusieurs excipients ou véhicules inertes, non toxiques, pharmaceutiquement acceptables.

17. Compositions pharmaceutiques selon la revendication 16 contenant au moins un principe actif selon l'une quelconque des revendications 1 à 13 utiles pour le traitement des troubles liés au système mélatoninergique.

## Patentansprüche

1. Verbindungen der Formel (I): 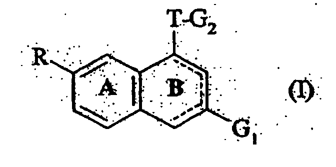 in der:
• die Ringe A und B gemeinsam eine Naphthalin-, 2,3-Dihydronaphthalin-, 1,4-Dihydronaphthalin-, 1,2-Dihydronaphthalin- oder Tetrahydronaphthalin-gruppe bilden,
• T eine geradkettige oder verzweigte (C₁-C₆)-Alkylenkette bedeutet, die gegebenenfalls durch eine oder mehrere geradkettige oder verzweigte (C₁-C₆)-Alkylgruppen, Hydroxygruppen, geradkettige oder verzweigte (C₁-C₆)-Alkoxygruppen, Carboxygruppen oder geradkettige oder verzweigte (C₁-C₆)-Alkoxycarbonylgruppen substituiert ist,
• R eine Gruppe R' oder eine Gruppe OR' bedeutet, worin R' eine geradkettige oder verzweigte (C₁-C₆)-Alkylgruppe oder geradkettige oder verzweigte (C₂-C₆)-Alkenylgruppe darstellt,
• G₁ eine gegebenenfalls substituierte Arylgruppe oder gegebenenfalls substituierte Heteroarylgruppe bedeutet,
• G₂ eine Gruppe bedeutet, ausgewählt aus: 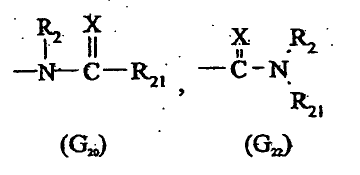
in denen:
- X ein Sauerstoffatom darstellt,
- R₂ ein Wasserstoffatom darstellt,
- R₂₁ eine geradkettige oder verzweigte, gegebenenfalls durch ein oder mehrere Halogenatome substituierte (C₁-C₆)-Alkylgruppe, geradkettige oder verzweigte (C₂-C₆)-Alkenylgruppe, geradkettige oder verzweigte (C₂-C₆)-Alkinylgruppe oder (C₃-C₇)-Cycloalkylgruppe bedeutet,
mit der Maßgabe, daß:
- wenn G₂ eine Gruppe G₂₂ darstellt, dann G₁ eine gegebenenfalls substituierte (von Pyridyl verschiedene) Heteroarylgruppe darstellt,
- wenn G₂ eine Gruppe G₂₀ bedeutet und G₁ von einer gegebenenfalls substituierten Heteroarylgruppe verschieden ist, dann T eine Methylenkette oder Ethylenkette bedeutet, die gegebenenfalls durch eine oder mehrere geradkettige oder verzweigte (C₁-C₆)-Alkylgruppen, Hydroxygruppen, geradkettige oder verzweigte (C₁-C₆)-Alkoxygruppen, Carboxygruppen oder geradkettige oder verzweigte (C₁-C₆)-Alkoxycarbonylgruppen substituiert ist,
der Begriff Aryl für eine Phenyl- oder Naphthylgruppe steht,
der Begriff Heteroaryl für eine gesättigte oder ungesättigte, mono- oder bicyclische Gruppe mit 4 bis 11 Kettengliedern steht, die 1 bis 4 Heteroatome ausgewählt aus Stickstoff, Sauerstoff und Schwefel aufweist,
wobei es sich versteht, daß:
- der Begriff gegebenenfalls substituiert im Hinblick auf die Begriffe Aryl-und Heteroarylgruppe bedeutet, daß diese Gruppen durch ein oder mehrere Halogenatome oder geradkettige oder verzweigte (C₁-C₆)-Alkylgruppen, geradkettige oder verzweigte (C₁-C₆)-Trihalogenalkylgruppen, Hydroxygruppen, geradkettige oder verzweigte (C₁-C₆)-Alkoxygruppen, Nitrogruppen, Aminogruppen (die gegebenenfalls durch eine oder zwei gleichartige oder verschiedenartige, geradkettige oder verzweigte (C₁-C₆)-Alkylgruppen substituiert sind), Cyanogruppen, Carboxygruppen, geradkettige oder verzweigte (C₁-C₆)-Alkylcarbonylgruppen oder Aminocarbonylgruppen (die gegebenenfalls durch eine oder zwei, gleichartige oder verschiedenartige, geradkettige oder verzweigte (C₁-C₆)-Alkylgruppen substituiert sind) substituiert sind,
deren Enantiomere, Diastereoisomere sowie deren Additionssalze mit einer pharmazeutisch annehmbaren Säure oder Base.

2. Verbindungen der Formel (I) nach Anspruch 1, worin:
• die Ringe A und B gemeinsam eine Naphthalingruppe bilden,
• T eine geradkettige oder verzweigte (C₁-C₆)-Alkylenkette bedeutet,
• R einen Rest R' oder eine Gruppe OR' bedeutet, worin R' eine geradkettige oder verzweigte (C₁-C₆)-Alkylgruppe oder eine geradkettige oder verzweigte (C₂-C₆)-Alkenylgruppe darstellt,
• G₁ eine gegebenenfalls substituierte Arylgruppe oder gegebenenfalls substituierte Heteroarylgruppe bedeutet,
• G₂ eine Gruppe bedeutet ausgewählt aus: 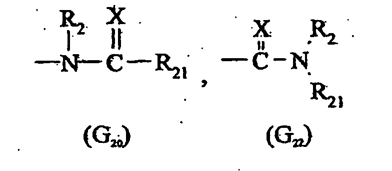 in denen:
- X ein Sauerstoffatom bedeutet,
- R₂ ein Wasserstoffatom bedeutet,
- R₂₁ eine geradkettige oder verzweigte, gebenenfalls durch ein oder mehrere Halogenatome substituierte (C₁-C₆)-Alkylgruppe, geradkettige oder verzweigte (C₂-C₆)-Alkenylgruppe, geradkettige oder verzweigte (C₂-C₆)-Alkinylgruppe oder (C₃-C₇)-Cycloalkylgruppe bedeutet,
deren Enantiomere, Diastereoisomere sowie deren Additionssalze mit einer pharmazeutisch annehmbaren Säure oder Base.

3. Verbindungen der Formel (I) nach Anspruch 1, worin:
• die Ringe A und B gemeinsam eine Tetrahydronaphthalingruppe bilden,
• T eine geradkettige oder verzweigte (C₁-C₆)-Alkylenkette bedeutet,
• R einen Rest R' oder eine Gruppe OR' bedeutet, worin R' eine geradkettige oder verzweigte (C₁-C₆)-Alkylgruppe oder geradkettige oder verzweigte (C₂-C₆)-Alkenylgruppe darstellt,
• G₁ eine gegebenenfalls substituierte Arylgruppe oder gegebenenfalls substituierte Heteroarylgruppe bedeutet,
• G₂ eine Gruppe bedeutet ausgewählt aus: 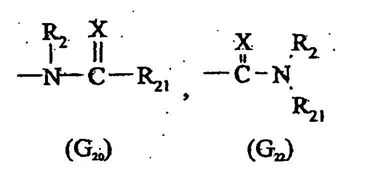 in denen:
- X ein Sauerstoffatom bedeutet,
- R₂ ein Wasserstoffatom bedeutet,
- R₂₁ eine geradkettige oder verzweigte, gebenenfalls durch ein oder mehrere Halogenatome substituierte (C₁-C₆)-Alkylgruppe, geradkettige oder verzweigte (C₂-C₆)-Alkenylgruppe, geradkettige oder verzweigte (C₂-C₆)-Alkinylgruppe oder (C₃-C₇)-Cycloalkylgruppe bedeutet,
deren Enantiomere, Diastereoisomere sowie deren Additionssalze mit einer pharmazeutisch annehmbaren Säure oder Base.

4. Verbindungen der Formel (I) nach Anspruch 1, worin T eine Alkylenkette mit 2 oder 3 Kohlenstoffatomen bedeutet, deren Enantiomere, Diastereoisomere sowie deren Additionssalze mit einer pharmazeutisch annehmbaren Säure oder Base.

5. Verbindungen der Formel (I) nach Anspruch 1, worin G₁ eine gegebenenfalls substituierte Arylgruppe bedeutet, deren Enantiomere, Diastereoisomere sowie deren Additionssalze mit einer pharmazeutisch annehmbaren Säure oder Base.

6. Verbindungen der Formel (I) nach Anspruch 1, worin G₁ eine gegebenenfalls substituierte Heteroarylgruppe bedeutet, deren Enantiomere, Diastereoisomere sowie deren Additionssalze mit einer pharmazeutisch annehmbaren Säure oder Base.

7. Verbindungen der Formel (I) nach Anspruch 1, worin R eine Gruppe R' bedeutet, worin R' eine geradkettige oder verzweigte (C₁-C₆)-Alkylgruppe oder geradkettige oder verzweigte (C₂-C₆)-Alkenylgruppe darstellt, deren Enantiomere, Diastereoisomere sowie deren Additionssalze mit einer pharmazeutisch annehmbaren Säure oder Base.

8. Verbindungen der Formel (I) nach Anspruch 1, worin R eine Gruppe OR' darstellt, worin R' eine geradkettige oder verzweigte (C₁-C₆)-Alkylgruppe oder eine geradkettige oder verzweigte (C₂-C₆)-Alkenylgruppe darstellt, deren Enantiomere, Diastereoisomere sowie deren Additionssalze mit einer pharmazeutisch annehmbaren Säure oder Base.

9. Verbindung der Formel (I) nach Anspruch 1, nämlich N-[2-(7-Methoxy-3-phenyl-1-naphthyl)-ethyl]-acetamid.

10. Verbindung der Formel (I) nach Anspruch 1, nämlich N-{2-[7-Methoxy-3-(3-trifluormethylphenyl)-1-naphthyl]-ethyl}-acetamid.

11. Verbindung der Formel (I) nach Anspruch 1, nämlich N-{2-[-7-Methoxy-3-(3-aminophenyl)-1-naphthyl]-ethyl}-acetamid, sowie dessen Additionssalze mit einer pharmazeutisch annehmbaren Säure oder Base.

12. Verbindungen der Formel (I) nach Anspruch 1, nämlich:
- N-{2-[3-(2-Furyl)-7-methoxy-1-naphthyl]-ethyl}-acetamid,
- N-{2-[7-Methoxy-3-(4-pyridyl)-1-naphthyl]-ethyl}-acetamid.

13. Verbindungen der Formel (I) nach Anspruch 1, nämlich:
- N-[2-(7-Methoxy-3-phenyl-1,2,3,4-tetrahydro-1-naphthyl)-ethyl]-acetamid,
- N-{2-[7-Methoxy-3-(3-trifluormethylphenyl)-1,2,3,4-tetrahydro-1-naphthyl]-ethyl}-acetamid.

14. Verfahren zur Herstellung der Verbindungen der Formel (I) nach Anspruch 1, **dadurch gekennzeichnet, daß** man als Ausgangsprodukt eine Verbindung der Formel (II/a) verwendet: 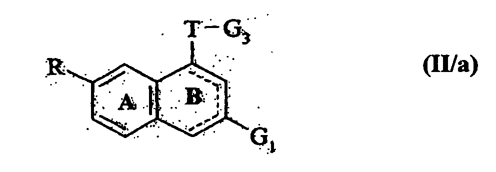 in der T, A, B, R und G₁ die bezüglich der Formel (I) angegebenen Bedeutungen besitzen und G₃ eine Gruppe -COOH oder -NH-R₂ bedeutet, worin R₂ die bezüglich der Formel (I) angegebenen Bedeutungen besitzt, welche man dann, wenn G₃ eine Gruppe -NH-R₂ darstellt,
a) mit einem Acylchlorid der Formel (III): 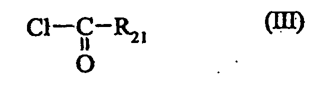 in der R₂₁ die bezüglich der Formel (I) angegebenen Bedeutungen besitzt,
oder mit dem entsprechenden (gemischten oder symmetrischen) Säureanhydrid umsetzt,
zur Bildung einer Verbindung der Formel (I/a): 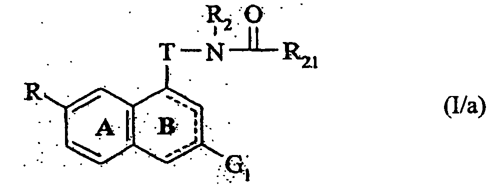 einem Sonderfall der Verbindungen der Formel (I), in der T, A, B, R, R₂, R₂₁
und G₁ die bezüglich der Formel (I) angegebenen Bedeutungen besitzen, oder
dann, wenn G₃ eine Carboxygruppe bedeutet, mit einer Verbindung der Formel (V): 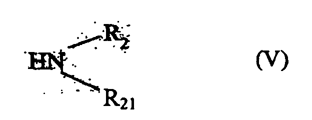 in der R₂ und R₂₁ die bezüglich der Formel (I) angegebenen Bedeutungen besitzen,
umsetzt, zur Bildung einer Verbindung der Formel (I/d): 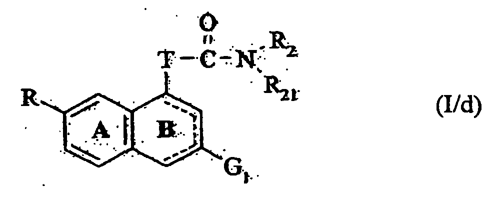 einem Sonderfall der Verbindungen der Formel (I), in der T, A, B, R, R₂, R₂₁ und G₁ die oben angegebenen Bedeutungen besitzen,
welche Verbindungen (I/a) und (I/d), welche die Gesamtheit der Verbindungen der Formel (I) bilden,
- gegebenenfalls mit Hilfe einer klassischen Reinigungsmethode gereinigt werden können,
- gegebenenfalls mit Hilfe einer klassischen Trennmethode in ihre Isomeren getrennt werden,
- gewünschtenfalls in ihre Additionssalze mit einer pharmazeutisch annehmbare Säure oder Base überführt werden.

15. Verfahren zur Herstellung der Verbindungen der Formel (I) nach Anspruch 1, **dadurch gekennzeichnet, daß** man als Ausgangsprodukt eine Verbindung der Formel (II/b) verwendet: 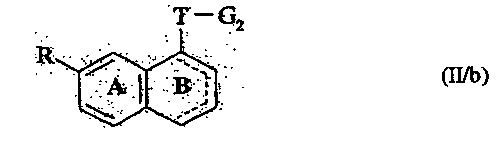 in der T, A, B, R und G₂ die bezüglich der Formel (I) angegebenen Bedeutungen besitzen,
welche man einer Acylierungsreaktion unterwirft unter Verwendung eines Acylchlorid-Reagens Cl-CO-G₁, worin G₁ die bezüglich der Formel (I) angegebenen Bedeutungen besitzt, zur Bildung einer Verbindung der Formel (II/c): 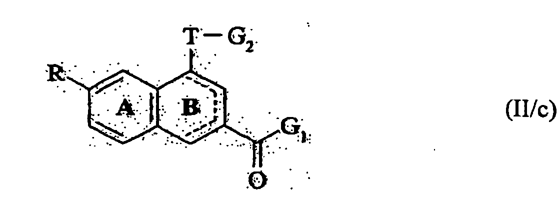 in der T, A, B, R, G₁ und G₂ die bezüglich der Formel (I) angegebenen Bedeutungen besitzen,
welche einer Baeyer-Villiger-Reaktion unterworfen werden kann zur Bildung einer Verbindung der Formel (I/g): 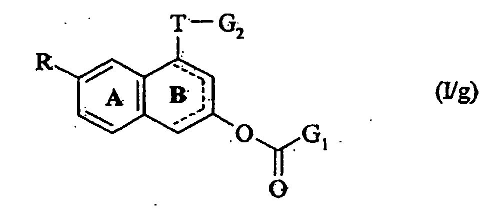 in der T, A, B, R, G₁ und G₂ die oben angegebenen Bedeutungen besitzen,
was durch eine Verseifungsreaktion zu einer Verbindung der Formel (VI) führt: 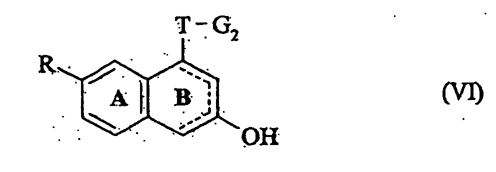 in der T, A, B, R und G₂ die bezüglich der Formel (I) angegebenen Bedeutungen besitzen,
von welcher Verbindung der Formel (VI) man die Hydroxylfunktion in eine Trifluormethansulfonatgruppe umwandelt unter Verwendung von beispielsweise Phenyl-bis(trifluormethan-sulfonimid) in basischem Medium, zur Bildung der Verbindung der Formel (VII): 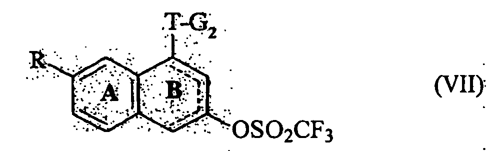 in der T, A, B, R und G₂ die oben angegebenen Bedeutungen besitzen,
welche man über eine durch ein geeignetes Palladium(0)-Derivat katalysierte Reaktion unter Verwendung eines Borsäurederivats (G₁B(OH)₂) oder eines Zinnderivats (G₁SnBu₃) als Reagens, worin G₁ die bezüglich der Formel (I) angegebenen Bedeutungen besitzt, in die Verbindung der Formel (I) umwandelt: 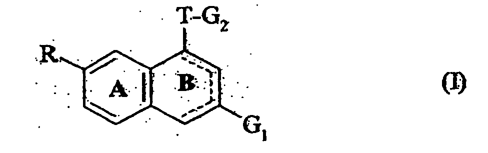 in der T, A, B, R, G₁ und G₂ die oben angegebenen Bedeutungen besitzen, welche Verbindungen der Formel (I)
- man gegebenenfalls mit Hilfe einer klassischen Reinigungsmethode reinigt,
- die man gegebenenfalls mit Hilfe einer klassischen Trennmethode in ihre Isomeren auftrennt,
- und gegebenenfalls in ihre Additionssalze mit einer pharmazeutisch annehmbaren Säure oder Base umwandelt,
welche Verbindungen der Formel (I) dann, wenn R eine Gruppe O-Alk darstellt (worin -Alk eine geradkettige oder verzweigte (C₁-C₆)-Alkylgruppe bedeutet), wenn dies mit dem an dem Molekül vorhandenen Substituenten verträglich ist, mit Bortribromid behandelt werden können zur Bildung der entsprechenden Hydroxyverbindung der Formel (VIII): 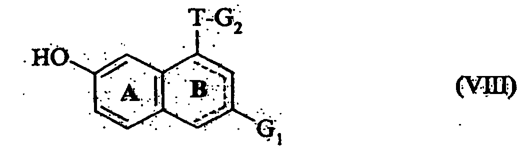 in der T, A, B, G₁ und G₂ die bezüglich der Formel (I) angegebenen Bedeutungen besitzen,
wobei man die Hydroxyfunktion der Verbindung (VIII):
- entweder in das Trifluormethansulfonat umwandeln kann zur Bildung einer Verbindung der Formel (IX): 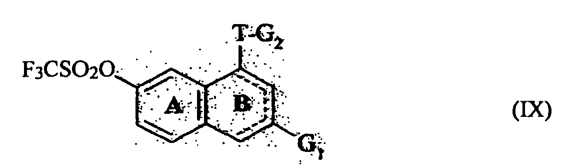 einem Sonderfall der Verbindungen der Formel (I), in der T, A, B, G₁ und G₂ die oben angegebenen Bedeutungen besitzen,
welche über eine durch ein Palladium(0)-Derivat katalysierte Reaktion unter Verwendung eines Borsäurederivats (R'B(OH)₂) oder eines Zinnderivats (R'SnBu₃), worin R' die bezüglich der Formel (I) angegebenen Bedeutungen besitzt, als Reagens die Bildung der Verbindung der Formel (I/i) ermöglicht: 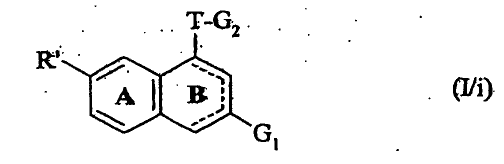 einem Sonderfall der Verbindungen der Formel (I), in der T, A, B, R', G₁ und G₂ die bezüglich der Formel (I) angegebenen Bedeutungen besitzen,
- oder der Einwirkung einer O-Substitutionsreaktion in basischem Medium unter Verwendung eines geeigneten Halogenderivats als Reagens unterworfen werden kann zur Bildung einer Verbindung der Formel (I/j): 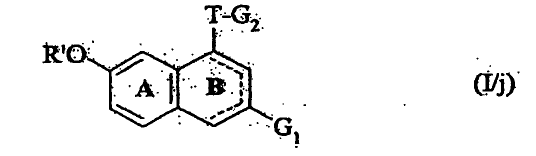 einem Sonderfall der Verbindungen der Formel (I), in der T, A, B, R', G₁ und G₂ die oben angegebenen Bedeutungen besitzen,
welche Verbindungen der Formel (I/i) und (I/j):
- man gegebenenfalls mit Hilfe einer klassischen Reinigungsmethode reinigt,
- man gegebenenfalls mit Hilfe einer klassischen Trennmethode in ihre Isomeren auftrennt,
- und man gegebenenfalls in ihre Additionssalze mit einer pharmazeutisch annehmbaren Säure oder Base überführt.

16. Pharmazeutische Zubereitungen enthaltend als Wirkstoff mindestens eine Verbindung nach einem der Ansprüche 1 bis 13 allein oder in Kombination mit einem oder mehreren inerten, nichttoxischen, pharmazeutisch annehmbaren Trägermaterialien oder Hilfsstoffen.

17. Pharmazeutische Zubereitungen nach Anspruch 16, enthaltend mindestens einen Wirkstoff nach einem der Ansprüche 1 bis 13 für die Behandlung von mit Störungen des melatoninergischen Systems verknüpften Störungen.

## Claims

1. Compounds of formula (I) : 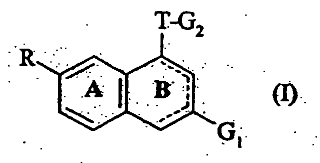 wherein :
• the A and B rings together form a naphthalene, 2,3-dihydronaphthalene, 1,4-dihydronaphthalene, 1,2-dihydronaphthalene or tetrahydronaphthalene group,
• T represents a linear or branched (C₁-C₆)alkylene chain optionally substituted by one or more linear or branched (C₁-C₆)alkyl, hydroxy, linear or branched (C₁-C₆)alkoxy, carboxy or linear or branched (C₁-C₆)alkoxycarbonyl groups,
• R represents a radical R' or a group OR', R' representing a linear or branched (C₁-C₆)alkyl or linear or branched (C₂-C₆)alkenyl group,
• G₁ represents an optionally substituted aryl or optionally substituted heteroaryl group,
• G₂ represents a group selected from : 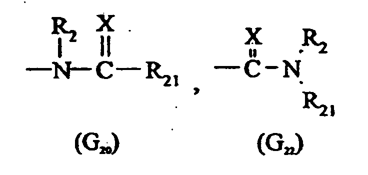
wherein:
- X represents an oxygen atom,
- R₂ represents a hydrogen atom,
- R₂₁ represents a linear or branched (C₁-C₆)alkyl group optionally substituted by one or more halogen atoms, a linear or branched (C₂-C₆)alkenyl group, a linear or branched (C₂-C₆)alkynyl group or a (C₃-C₇)cycloalkyl group,
with the proviso that:
- when G₂ represents a group G₂₂, then G₁ represents an optionally substituted heteroaryl group (other than a pyridyl group),
- when G₂ represents a group G₂₀ and G₁ is other than an optionally substituted heteroaryl group, then T represents a methylene or ethylene chain optionally substituted by one or more linear or branched (C₁-C₆)alkyl, hydroxy, linear or branched (C₁-C₆)alkoxy, carboxy or linear or branched (C₁-C₆)alkoxycarbonyl groups,
the term "aryl" denoting a phenyl or naphthyl group,
the term "heteroaryl" denoting a mono- or bi-cyclic group having from 4 to 11 ring members, being saturated or unsaturated and containing from 1 to 4 hetero atoms selected from nitrogen, oxygen and sulphur,
it being understood that :
- the term "optionally substituted" applied to the terms "aryl" and "heteroaryl" means that those groups are substituted by one or more halogen atoms, or linear or branched (C₁-C₆)alkyl, linear or branched trihalo(C₁-C₆)alkyl, hydroxy, linear or branched (C₁-C₆)alkoxy or nitro groups or amino groups (optionally substituted by one or two identical or different linear or branched (C₁-C₆)alkyl groups) or cyano, carboxy or linear or branched (C₁-C₆)alkylcarbonyl groups or aminocarbonyl groups (optionally substituted by one or two identical or different linear or branched (C₁-C₆)alkyl groups),
their enantiomers, diastereoisomers, and addition salts thereof with a pharmaceutically acceptable acid or base.

2. Compounds of formula (I) according to claim 1, wherein :
• the A and B rings together form a naphthalene group,
• T represents a linear or branched (C₁-C₆)alkylene chain,
• R represents a radical R' or a group OR', R' representing a linear or branched (C₁-C₆)alkyl or linear or branched (C₂-C₆)alkenyl group,
• G₁ represents an optionally substituted aryl or optionally substituted heteroaryl group,
• G₂ represents a group selected from : 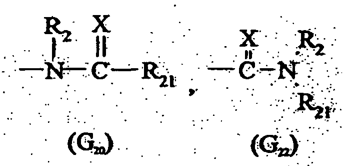
wherein :
- X represents an oxygen atom,
- R₂ represents a hydrogen atom,
- R₂₁ represents a linear or branched (C₁-C₆)alkyl group optionally substituted by one or more halogen atoms, a linear or branched (C₂-C₆)alkenyl group, a linear or branched (C₂-C₆)alkynyl group or a (C₃-C₇)cycloalkyl group,
their enantiomers, diastereoisomers, and addition salts thereof with a pharmaceutically acceptable acid or base.

3. Compounds of formula (I) according to claim 1, wherein :
• the A and B rings together form a tetrahydronaphthalene group,
• T represents a linear or branched (C₁-C₆)alkylene chain,
• R represents a radical R' or a group OR', R' representing a linear or branched (C₁-C₆)alkyl or linear or branched (C₂-C₆)alkenyl group,
• G₁ represents an optionally substituted aryl or optionally substituted heteroaryl group,
• G₂ represents a group selected from : 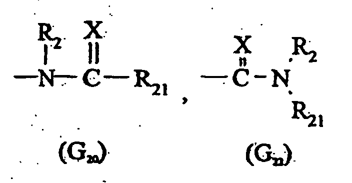
wherein:
- X represents an oxygen atom,
- R₂ represents a hydrogen atom,
- R₂₁ represents a linear or branched (C₁-C₆)alkyl group optionally substituted by one or more halogen atoms, a linear or branched (C₂-C₆)alkenyl group, a linear or branched (C₂-C₆)alkynyl group or a (C₃-C₇)cycloalkyl group,
their enantiomers, diastereoisomers, and addition salts thereof with a pharmaceutically acceptable acid or base.

4. Compounds of formula (I) according to claim 1, wherein T represents an alkylene chain having 2 or 3 carbon atoms, their enantiomers, diastereoisomers, and addition salts thereof with a pharmaceutically acceptable acid or base.

5. Compounds of formula (I) according to claim 1, wherein G₁ represents an optionally substituted aryl group, their enantiomers, diastereoisomers, and addition salts thereof with a pharmaceutically acceptable acid or base.

6. Compounds of formula (I) according to claim 1, wherein G₁ represents an optionally substituted heteroaryl group, their enantiomers, diastereoisomers, and addition salts thereof with a pharmaceutically acceptable acid or base.

7. Compounds of formula (I) according to claim 1, wherein R represents a group R', R' being a linear or branched (C₁-C₆)alkyl or linear or branched (C₂-C₆)alkenyl group, their enantiomers, diastereoisomers, and addition salts thereof with a pharmaceutically acceptable acid or base.

8. Compounds of formula (I) according to claim 1, wherein R represents a group OR', R' being a linear or branched (C₁-C₆)alkyl or linear or branched (C₂-C₆)alkenyl group, their enantiomers, diastereoisomers, and addition salts thereof with a pharmaceutically acceptable acid or base.

9. Compound of formula (I) according to claim 1, which is N-[2-(7-methoxy-3-phenyl-1-naphthyl)ethyl]acetamide.

10. Compound of formula (I) according to claim 1, which is N-{2-[7-methoxy-3-(3-trifluoromethylphenyl)-1-naphthyl]ethyl}acetamide.

11. Compound of formula (I) according to claim 1, which is N-{2-[7-methoxy-3-(3-aminophenyl)-1-naphthyl]ethyl}acetamide, and its addition salts with a pharmaceutically acceptable acid or base.

12. Compounds of formula (I) according to claim 1, which are :
- N-{2-[3-(2-furyl)-7-methoxy-1-naphthyl]ethyl}acetamide,
- N-{2-[7-methoxy-3-(4-pyridyl)-1-naphthyl]ethyl}acetamide.

13. Compounds of formula (I) according to claim 1, which are :
- N-[2-(7-methoxy-3-phenyl-1,2,3,4-tetrahydro-1-naphthyl)ethyl]acetamide,
- N-{2-[7-methoxy-3-(3-trifluoromethylphenyl)-1,2,3,4-tetrahydro-1-naphthyl]-ethyl}acetamide.

14. Process for the preparation of compounds of formula (I) according to claim 1, **characterised in that** there is used as starting material a compound of formula (II/a) : 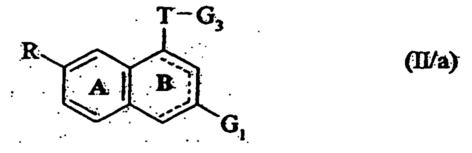 wherein T, A, B, R and G₁ are as defined for formula (I) and G₃ represents a group -COOH or -NH-R₂, R₂ being as defined for formula (I),
which, when G₃ represents a group -NH-R₂, is reacted with
a) an acyl chloride of formula (III) : 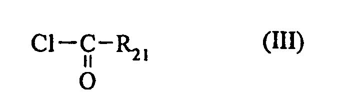
wherein R₂₁ is as defined for formula (I),
or with a corresponding acid anhydride (mixed or symmetrical),
to yield a compound of formula (I/a) : 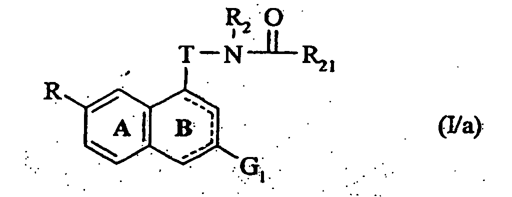 which is a particular case of the compounds of formula (I) wherein T, A, B, R, R₂, R₂₁ and G₁ are as defined for formula (I),
or,
when G₃ represents a carboxy group, is reacted with a compound of formula (V) : 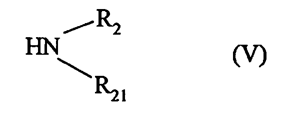 wherein R₂ and R₂₁ are as defined for formula (I),
to yield a compound of formula (I/d) : 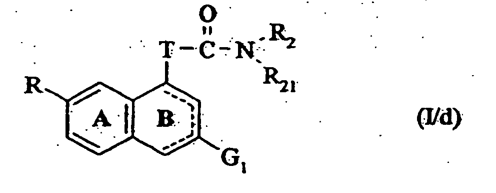 which is a particular case of the compounds of formula (I) wherein T, A, B, R, R₂, R₂₁ and G₁ are as defined hereinbefore,
which compounds (I/a) and (I/d), constituting the totality of the compounds of formula (I),
- can be purified, if necessary, according to a conventional purification technique,
- are separated, where appropriate, into their isomers according to a conventional separation technique,
- are converted, if desired, into addition salts thereof with a pharmaceutically acceptable acid or base.

15. Process for the preparation of compounds of formula (I) according to claim 1, **characterised in that** there is used as starting material a compound of formula (II/b) : 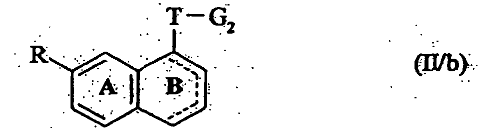 wherein T, A, B, R and G₂ are as defined for formula (I),
which is subjected to an acylation reaction using as reagent an acyl chloride Cl-CO-G₁, G₁ being as defined for formula (I), to yield a compound of formula (II/c) : 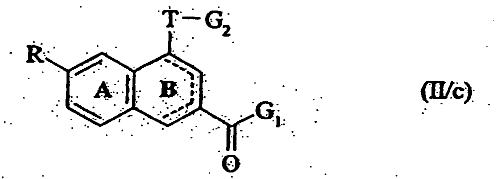 wherein T, A, B, R, G₁ and G₂ are as defined for formula (I),
which can be subjected to a Baeyer-Villiger reaction to yield a compound of formula (I/g) : 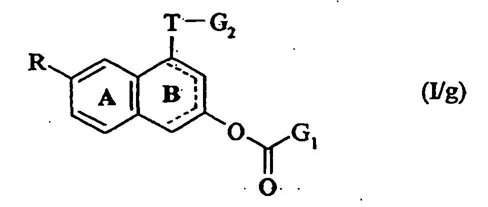 wherein T, A, B, R, G₁ and G₂ are as defined hereinbefore,
which, by a hydrolysis reaction, yields a compound of formula (VI) : 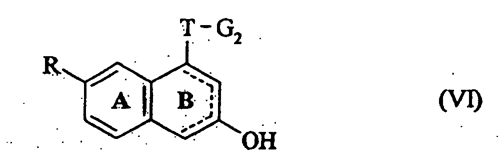 wherein T, A, B, R and G₂ are as defined for formula (I),
the hydroxyl function of which compound of formula (VI) is converted to trifluoromethanesulphonate, using, for example, phenyl bis(trifluoromethanesulphonimide) in a basic medium, to yield a compound of formula (VII) : 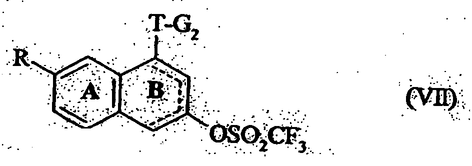 wherein T, A, B, R and G₂ are as defined hereinbefore,
which can be converted, *via* the intermediary of a reaction catalysed by a suitable palladium(0) compound using as reagent a boric acid compound (G₁B(OH)₂) or a tin compound (G₁SnBu₃), with G₁ being as defined for formula (I), to a compound of formula (I) : 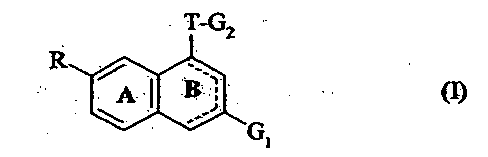 wherein T, A, B, R, G₁ and G₂ are as defined hereinbefore,
which compounds of formula (I)
- are purified, if necessary, according to a conventional purification technique,
- are separated, where appropriate, into their isomers according to a conventional separation technique, and
- are converted, if desired, into addition salts thereof with a pharmaceutically acceptable acid or base,
which compounds of formula (I), when R represents an O-Alk group (-Alk representing a linear or branched (C₁-C₆)alkyl group), can, when that is compatible with the substituents present on the molecule, be treated with boron tribromide to yield a corresponding hydroxylated compound of formula (VIII) : 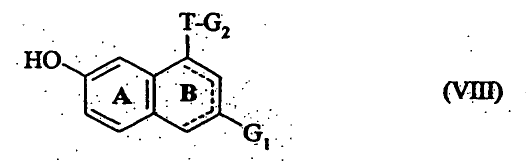 wherein T, A, B, G₁ and G₂ are as defined for formula (I),
the hydroxyl function of which compound (VIII) can :
- either be converted to trifluoromethanesulphonate to yield a compound of formula (IX) : 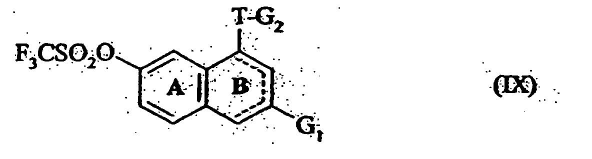 which is a particular case of the compounds of formula (I) wherein T, A, B, G₁ and G₂ are as defined hereinbefore,
which, *via* the intermediary of a reaction catalysed by a palladium(0) compound using as reagent a boric acid compound (R'B(OH)₂) or a tin compound (R'SnBu₃) wherein R' is as defined for formula (I), makes it possible to obtain a compound of formula (I/i) : 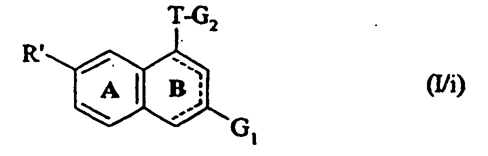 which is a particular case of the compounds of formula (I) wherein T, A, B, R', G₁ and G₂ are as defined for formula (I),
- or be subjected to an O-substitution reaction, in a basic medium, using the appropriate halogenated compound as reagent, to yield a compound of formula (I/j) : 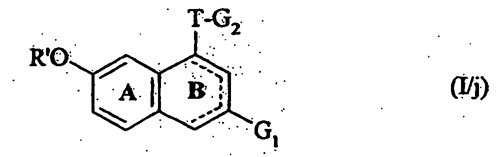 which is a particular case of the compounds of formula (I) wherein T, A, B, R', G₁ and G₂ are as defined hereinbefore,
which compounds of formulae (I/i) and (I/j) :
- are purified, if necessary, according to a conventional purification technique,
- are separated, where appropriate, into their isomers according to a conventional separation technique,
- and are converted, if desired, into addition salts thereof with a pharmaceutically acceptable acid or base.

16. Pharmaceutical compositions comprising as active ingredient at least one compound according to any one of claims 1 to 13 on its own or in combination with one or more inert, non-toxic, pharmaceutically acceptable excipients or carriers.

17. Pharmaceutical compositions according to claim 16 comprising at least one active ingredient according to any one of claims 1 to 13 for use in the treatment of disorders associated with the melatoninergic system.
